# EUROPEAN PATENT SPECIFICATION

(11) **EP 2 341 774 B1**
(45) Date of publication and mention of the grant of the patent: **04.12.2013**
(21) Application number: 09822452.0
(22) Date of filing: 15.10.2009
(51) Int. Cl.: A61K 31/44, A01N 43/42, A61P 35/00

(54) **TREATMENT OF NEUROBLASTOMA WITH MULTI-ARM POLYMERIC CONJUGATES OF 7-ETHYL-10-HYDROXYCAMPTOTHECIN**
BEHANDLUNG VON NEUROBLASTOMEN MIT MEHRARMIGEN POLYMERKONJUGATEN AUS 7-ETHYL-10-HYDROXYCAMPTOTHECIN
TRAITEMENT DE NEUROBLASTOMES AVEC DES CONJUGUÉS POLYMÈRES MULTI-BRAS DE 7-ÉTHYL-10-HYDROXYCAMPTOTHÉCINE

(30) Priority: 21.10.2008 US 107175 P; 17.04.2009 US 170285 P
(43) Date of publication of application: 13.07.2011
(73) Proprietor: Belrose Pharma Inc., Princeton, NJ 08540 (US)
(72) Inventor: PASTORINO, Fabio, I-16031 Bogliasco (Genoa) (IT); PONZONI, Mirco, I-16148 Genoa (IT)
(74) Representative: Jansen, Cornelis Marinus
(86) International application number: PCT/US2009/060765
(87) International publication number: WO 2010/048018

(56) References cited:
- WO-A2-2007/092646
- US-A1- 2005 112 088
- US-A1- 2007 197 575
- US-B1- 6 281 223
- SAPRA PUJA ET AL: "EZN-2208, a novel polyethyleneglycol-SN38 conjugate, has potent antitumor activity in a panel of human tumor xenografts", PROCEEDINGS OF THE ANNUAL MEETING OF THE AMERICAN ASSOCIATION FOR CANCER RESEARCH; 98TH ANNUAL MEETING OF THE AMERICAN-ASSOCIATION-FOR- CANCER-RESEARCH;LOS ANGELES, CA, USA; APRIL 14 -18, 2007, AMERICAN ASSOCIATION FOR CANCER RESEARCH, US, vol. 48, 1 April 2007 (2007-04-01), pages 1-2, XP008133384, ISSN: 0197-016X
- SAPRA P ET AL: "EZN-2208, A NOVEL POLYETHYLENEGLYCOL-SN38 CONJUGATE, THERAPY RESULTS IN CURES OF ANIMALS BEARING AGGRESSIVE NON-HODGKIN'S LYMPHOMA MODELS", HAEMATOLOGICA-THE HEMATOLOGY JOURNAL, vol. 93, no. Suppl. 1, June 2008 (2008-06) , page 114, XP002669309, 13TH CONGRESS OF THE EUROPEAN-HEMATOLOGY-ASSOCIATION; COPENHAGEN, DENMARK; JUNE 12 -15, 2008
- PUJA SAPRA ET AL: "Novel delivery of SN38 markedly inhibits tumor growth in xenografts, including a camptothecin-11-refractory model", CLINICAL CANCER RESEARCH, THE AMERICAN ASSOCIATION FOR CANCER RESEARCH, US, vol. 14, no. 6, 15 March 2008 (2008-03-15) , pages 1888-1896, XP002624712, ISSN: 1078-0432, DOI: 10.1158/1078-0432.CCR-07-4456

## Description

### CROSS-REFERENCE TO RELATED APPLICATION

This application claims the benefit of priority from U.S. Provisional Patent Application Serial Nos. 61/107,175 filed October 21, 2008 and 61/170,285 filed April 17, 2009.

### FIELD OF THE INVENTION

The present invention relates to polymeric prodrugs of 7-ethyl-10-hydroxycamptothecin, in particular, polyethylene glycol conjugates of 7-ethyl-10-hydroxy-camptothecin for use in a method of treatment of neuroblastoma.

### BACKGROUND OF THE INVENTION

Neuroblastoma is a cancer that develops from nerve tissue. Neuroblastoma is a solid tumor and commonly occurs in infants and children younger than 5 years, though it may rarely occur in older children and adults. Most neuroblastoma starts in and around the adrenal glands. Neuroblastoma may also begin in the abdomen, and in nerve tissue in the neck, chest, and pelvis, where nerve cells are present. The cancer often spreads to other parts of the body, such as the lymph nodes, bones, bone marrow, eyes, liver, skin and the tissue that surrounds the spinal cord.

Neuroblastoma is the second most common solid tumor in childhood. In the advanced stage of the disease, treatment of neuroblastoma is successful in less than a half of patients. The effective treatment of neuroblastoma, either at advanced stage or earlier stage of minimal residual disease, remains indeed one of the major challenges in pediatric oncology. The 5 year survival for the metastatic disease is still less than 60% and, consequently, novel therapeutic approaches are needed.

In an attempt to provide earlier diagnosis and treatment, screening infants for neuroblastoma was undertaken, but not helpful. In most case, neuroblasts (immature nerve cells) found by the screening disappear or mature into a benign tumor.

At present, treatment of neuroblastoma typically employs a combination of the standard anticancer agents, such as cyclophosphamide (ifosfamide), cisplatin (carboplatin), vincristine, doxorubicin, etoposide, teniposide, topotecan and melphalan. Unfortunately, neuroblastoma is commonly resistant to such conventional anticancer agents, and the cancer relapses after completion of treatment. Thus, there remains a longstanding need for alternative treatments for neuroblastoma. The present invention addresses this need.

WO2007/092646 discloses the use of PEG-SN38 conjugates for the treatment of solid tumors, lymphomas, small cell lung cancer, acute lymphocytic leukemia, pancreatic cancer, glioblastoma without mentioning neuroblastoma.

Sapra P. et al. (Proceedings of the Annual Meeting of the American Association for Cancer Research (2007), 48, 1 - 2) show the PEG-SN38 conjugate EZN-2208 and its efficacy in xenograft models of breast, colorectal and pancreatic cancer.

Sapra P. et al. (The Hematology Journal (Jun 2008), 93 (Suppl. 1), 114) demonstrate a successful use of the PEG-SN38 conjugate EZN-2208 in a xenograft model of non-Hodgkin's lymphoma.

Sapra P. et al. (Clinical Cancer Research (2008), 14(6), 1888 - 1896) report the efficacy of the PEG-SN38 conjugate EZN-2208 efficacy in breast, pancreatic or colon carcinoma refractory to CPT-11 treatment.

US2005/112088 discloses conjugates comprising a pentaerythritol core and anti-cancer drugs (e.g. camptothecins) for use in the treatment of various cancers, e.g. neuroblastoma.

### SUMMARY OF THE INVENTION

In one aspect of the present invention, compounds of formula (I) below are provided for use in a method of treating neuroblastoma in a mammal. The treatment includes administering an effective amount of a compound of Formula (I): 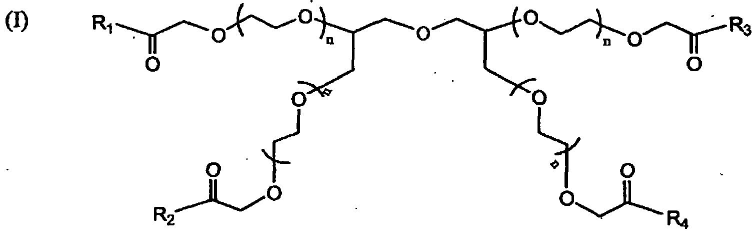 wherein
R₁, R₂, R₃ and R₄ are independently OH or 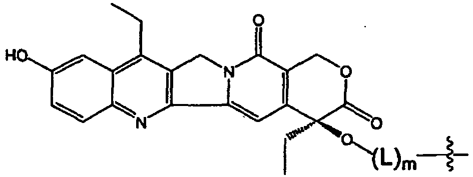 wherein
L is a bifunctional linker, and each L is the same or different when (m) is equal to or greater than 2;
(m) is a positive integer; and
(n) is a positive integer;
provided that R₁, R₂, R₃ and R₄ are not all OH;
or a pharmaceutically acceptable salt thereof to the mammal.

In one particular aspect of the invention, the polymeric prodrugs of 7-ethyl-10-hydroxycamptothecin for use in the treatment described herein employ four-arm PEG-7-ethyl-10-hydroxycamptothecin conjugates having the structure of 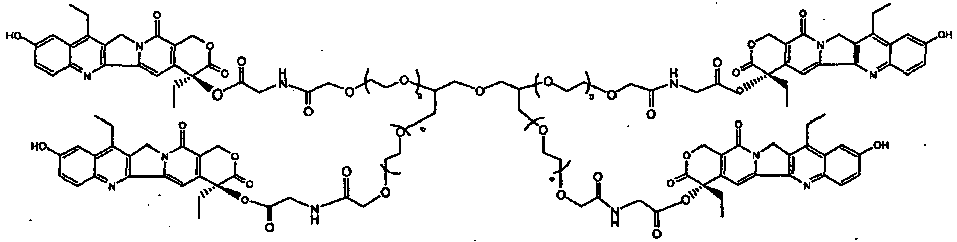 wherein (n) is from about 28 to about 341, preferably from about 114 to about 239, and more preferably about 227.

Simply by way of example, the above provided method of the invention is conducted wherein the compound of Formula (I) is administered in amounts of from about 0.5 mg/m² body surface/dose to about 50 mg/m² body surface/dose, and more particularly, wherein the compound of Formula (I) is administered in amounts of from about 1 mg/m² body surface/dose to about 18 mg/m² body surface/dose, and even more particularly, wherein the compound of Formula (I) is administered according to a protocol of from about 1.25 mg/m² body surface/dose to about 16.5 mg/m² body surface/dose given weekly for three weeks, followed by 1 week without treatment. In certain embodiments, the amount administered weekly is about 5 mg/m² body surface/dose.

In a further aspect, the present invention provides compounds of formula (I) for use in a method of treating neuroblastoma that is resistant or refractory to conventional anticancer methods, including chemotherapy. In one particular aspect, the treatment is effective for cancers resistant or refractory to camptothecin (CPT) or CPT-11 associated therapy. Alternatively, the present invention provides compounds of formula (I) for use in a method of treating neuroblastoma showing topoisomerase I mediated resistance or refractory phenomenon. In still alternative aspect, the present invention provides compounds of formula (I) for use in a method of treating neuroblastoma resistant or refractory to therapies associated with administration of polymeric prodrug forms of CPT or CPT-11 such as polyethylene glycol conjugates of CPT or CPT-11.

The polymeric prodrugs of 7-ethyl-10-hydroxycamptothecin according to the present invention are effective to treat neuroblastoma that is resistant or refractory at the onset of treatment or at a subsequent round of therapy. The present invention allows treatment of refractory neuroblastoma that is sensitive to CPT-11, i.e., which appears to be inhibited in the first round of treatment, but becomes resistant to treatment in the second or subsequent rounds of therapy. The polymeric prodrugs of 7-ethyl-10-hydroxycamptothecin can be further effective for treatment of recurring neuroblastoma after treatment is discontinued.

One advantage of the present invention is that patients can be treated concurrently or sequentially with an effective amount of the polymeric prodrugs of 7-ethyl-10-hydroxy-camptothecin in combination with another anti-cancer therapeutic agent for synergistic benefit.

Yet another advantage of the present invention is that the prodrugs described herein have reduced the toxicity and/or overcome other difficulties encountered during therapy, when compared to prior art anticancer agents. Non-hematological toxicities associated with the present invention are manageable and transient compared to the treatment associated with conventional anticancer agents. For example, the commonly used agent doxorubicin causes cardiotoxicity. The platinum-based anticancer agents (e.g., cisplatin, carboplatin, etc.) used in the treatment of neuroblastoma are known to cause kidney damage. See Cancer Principles and Practice, DeVita et al., p384-385. Therapies associated with conventional anticancer agents also cause bone marrow suppression such as leucopenia, neutropenia and/or thrombocytopenia.

On the other hand, the treatment according to the present invention uses relatively non-myelosuppressive dosages, in part, because the polymeric prodrugs prevent premature excretion of the active agent, 7-ethyl-10-hydroxycamptothecin. Sufficient amounts of the active agent can be released from the polymeric prodrugs and available in the body to exert therapeutic effects. The polymeric forms also eliminate or significantly reduce immune response. The compounds used in the present invention can be given safely to the patients. The compounds used in the present invention can be administered in combination with other anticancer drugs, either concurrently or sequentially. The present invention can be also performed with other types of treatments, i.e., radiotherapy.

Advantages will be apparent from the following description and drawings.

For purposes of the present invention, the term "residue" shall be understood to mean that portion of a compound, to which it refers, e.g., 7-ethyl-10-hydroxycamptothecin, amino acid, etc. that remains after it has undergone a substitution reaction with another compound.

For purposes of the present invention, the term "polymeric containing residue" or "PEG residue" shall each be understood to mean that portion of the polymer or PEG which remains after it has undergone a reaction with, e.g., an amino acid, 7-ethyl-10-hydroxycamptothecin-containing compounds.

For purposes of the present invention, the term "alkyl" refers to a saturated aliphatic hydrocarbon, including straight-chain, branched-chain, and cyclic alkyl groups. The term "alkyl" also includes alkyl-thio-alkyl, alkoxyalkyl, cycloalkylalkyl, heterocycloalkyl, and C₁₋₆ alkylcarbonylalkyl groups. Preferably, the alkyl group has 1 to 12 carbons. More preferably, it is a lower alkyl of from about 1 to 7 carbons, yet more preferably about 1 to 4 carbons. The alkyl group can be substituted or unsubstituted. When substituted, the substituted group(s) preferably include halo, oxy, azido, nitro, cyano, alkyl, alkoxy, alkyl-thio, alkyl-thio-alkyl, alkoxyalkyl, alkylamino, trihalomethyl, hydroxyl, mercapto, hydroxy, cyano, alkylsilyl, cycloalkyl, cycloalkylalkyl, heterocycloalkyl, heteroaryl, alkenyl, alkynyl, C₁₋₆ hydrocarbonyl, aryl, and amino groups.

For purposes of the present invention, the term "substituted" refers to adding or replacing one or more atoms contained within a functional group or compound with one of the moieties from the group of halo, oxy, azido, nitro, cyano, alkyl, alkoxy, alkyl-thio, alkyl-thio-alkyl, alkoxyalkyl, alkylamino, trihalomethyl, hydroxyl, mercapto, hydroxy, cyano, alkylsilyl, cycloalkyl, cycloalkylalkyl, heterocycloalkyl, heteroaryl, alkenyl, alkynyl, C₁₋₆ alkylcarbonylalkyl, aryl, and amino groups.

For purposes of the present invention, the term "alkenyl" refers to groups containing at least one carbon-carbon double bond, including straight-chain, branched-chain, and cyclic groups. Preferably, the alkenyl group has about 2 to 12 carbons. More preferably, it is a lower alkenyl of from about 2 to 7 carbons, yet more preferably about 2 to 4 carbons. The alkenyl group can be substituted or unsubstituted. When substituted the substituted group(s) include halo, oxy, azido, nitro, cyano, alkyl, alkoxy, alkyl-thio, alkyl-thio-alkyl, alkoxyakyl, alkylamino, trihalomethyl, hydroxyl, mercapto, hydroxy, cyano, alkylsilyl, cycloalkyl, cycloalkylalkyl, heterocycloalkyl, heteroaryl, alkenyl, alkynyl, C₁₋₆ hydrocarbonyl, aryl, and amino groups.

For purposes of the present invention, the term "alkynyl" refers to groups containing at least one carbon-carbon triple bond, including straight-chain, branched-chain, and cyclic groups. Preferably, the alkynyl group has about 2 to 12 carbons. More preferably, it is a lower alkynyl of from about 2 to 7 carbons, yet more preferably about 2 to 4 carbons. The alkynyl group can be substituted or unsubstituted. When substituted the substituted group(s) include halo, oxy, azido, nitro, cyano, alkyl, alkoxy, alkyl-thio, alkyl-thio-alkyl, alkoxyalkyl, alkylamino, trihalomethyl, hydroxyl, mercapto, hydroxy, cyano, alkylsilyl, cycloalkyl, cycloalkylalkyl, heterocycloalkyl, heteroaryl, alkenyl, alkynyl, C₁₋₆ hydrocarbonyl, aryl, and amino groups. Examples of "alkynyl" include propargyl, propyne, and 3-hexyne.

For purposes of the present invention, the term "aryl" refers to an aromatic hydrocarbon ring system containing at least one aromatic ring. The aromatic ring can optionally be fused of otherwise attached to other aromatic hydrocarbon rings or non-aromatic hydrocarbon rings. Examples of aryl groups include, for example, phenyl, naphthyl, 1,2,3,4-tetrahydronaphthalene and biphenyl. Preferred examples of aryl groups include phenyl and naphthyl.

For purposes of the present invention, the term "cycloalkyl" refers to a C₃₋₈ cyclic hydrocarbon. Examples of cycloalkyl include cyclopropyl, cyclobutyl, cyclopentyl, cyclohexyl, cycloheptyl and cyclooctyl.

For purposes of the present invention, the term "cycloalkenyl" refers to a C₃₋₈ cyclic hydrocarbon containing at least one carbon-carbon double bond. Examples of cycloalkenyl include cyclopentenyl, cyclopentadienyl, cyclohexenyl, 1,3-cyclohexadienyl, cycloheptenyl, cycloheptatrienyl, and cyclooctenyl.

For purposes of the present invention, the term "cycloalkylalkyl" refers to an alklyl group substituted with a C₃₋₈ cycloalkyl group. Examples of cycloalkylalkyl groups include cyclopropylmethyl and cyclopentylethyl.

For purposes of the present invention, the term "alkoxy" refers to an alkyl group of indicated number of carbon atoms attached to the parent molecular moiety through an oxygen bridge: Examples of alkoxy groups include, for example, methoxy, ethoxy, propoxy and isopropoxy.

For purposes of the present invention, an "alkylaryl" group refers to an aryl group substituted with an alkyl group.

For purposes of the present invention, an "aralkyl" group refers to an alkyl group substituted with an aryl group.

For purposes of the present invention, the term "alkoxyalkyl" group refers to an alkyl group substituted with an alkloxy group.

For purposes of the present invention, the term "amino" refers to a nitrogen containing group as is known in the art derived from ammonia by the replacement of one or more hydrogen radicals by organic radicals. For example, the terms "acylamino" and "alkylamino" refer to specific N-substituted organic radicals with acyl and alkyl substituent groups respectively.

For purposes of the present invention, the term "halogen' or "halo" refers to fluorine, chlorine, bromine, and iodine.

For purposes of the present invention, the term "heteroatom" refers to nitrogen, oxygen, and sulfur.

For purposes of the present invention, the term "heterocycloalkyl" refers to a non-aromatic ring system containing at least one heteroatom selected from nitrogen, oxygen, and sulfur. The heterocycloalkyl ring can be optionally fused to or otherwise attached to other heterocycloalkyl rings and/or non-aromatic hydrocarbon rings. Preferred heterocycloalkyl groups have from 3 to 7 members. Examples of heterocycloalkyl groups include, for example, piperazine, morpholine, piperidine, tetrahydrofuran, pyrrolidine, and pyrazole. Preferred heterocycloalkyl groups include piperidinyl, piperazinyl, morpholinyl, and pyrrolidinyl.

For purposes of the present invention, the term "heteroaryl" refers to an aromatic ring system containing at least one heteroatom selected from nitrogen, oxygen, and sulfur. The heteroaryl ring can be fused or otherwise attached to one or more heteroaryl rings, aromatic or non-aromatic hydrocarbon rings or heterocycloalkyl rings. Examples of heteroaryl groups include, for example, pyridine, furan, thiophene, 5,6,7,8-tetrahydroisoquinoline and pyrimidine. Preferred examples of heteroaryl groups include thienyl, benzothienyl, pyridyl, quinolyl, pyrazinyl, pyrimidyl, imidazolyl, benzimidazolyl, furanyl, benzofuranyl, thiazolyl, benzothiazolyl, isoxazolyl, oxadiazolyl, isothiazolyl, benzisothiazolyl, triazolyl, tetrazolyl, pyrrolyl, indolyl, pyrazolyl, and benzopyrazolyl.

In some embodiments, substituted alkyls include carboxyalkyls, aminoalkyls, dialkylaminos, hydroxyalkyls and mercaptoalkyls; substituted alkenyls include carboxyalkenyls, aminoalkenyls, dialkenylaminos, hydroxyalkenyls and mercaptoalkenyls; substituted alkynyls include carboxyalkynyls, aminoalkynyls, dialkynylaminos, hydroxyalkynyls and mercaptoalkynyls; substituted cycloalkyls include moieties such as 4-chlorocyclohexyl; aryls include moieties such as napthyl; substituted aryls include moieties such as 3-bromo phenyl; aralkyls include moieties such as tolyl; heteroalkyls include moieties such as ethylthiophene; substituted heteroalkyls include moieties such as 3-methoxy-thiophene; alkoxy includes moieties such as methoxy; and phenoxy includes moieties such as 3-nitrophenoxy.

For purposes of the present invention, "positive integer" shall be understood to include an integer equal to or greater than 1 and as will be understood by those of ordinary skill to be within the realm of reasonableness by the artisan of ordinary skill.

For purposes of the present invention, the term "linked" shall be understood to include covalent (preferably) or noncovalent attachment of one group to another, i.e., as a result of a chemical reaction.

The terms "effective amounts" and "sufficient amounts" for purposes of the present invention shall mean an amount which achieves a desired effect or therapeutic effect as such effect is understood by those of ordinary skill in the art. An effective amount for each mammal or human patient to be treated is readily determined by the artisan in a range that provides a desired clinical response while avoiding undesirable effects that are inconsistent with good practice. Dose ranges are provided hereinbelow.

For purposes of the present invention, the terms "cancer" and "tumor" are used interchangeably, unless otherwise indicated. "Cancer" encompasses malignant and/or metastatic cancer, unless otherwise indicated.

### BRIEF DESCRIPTION OF THE DRAWINGS

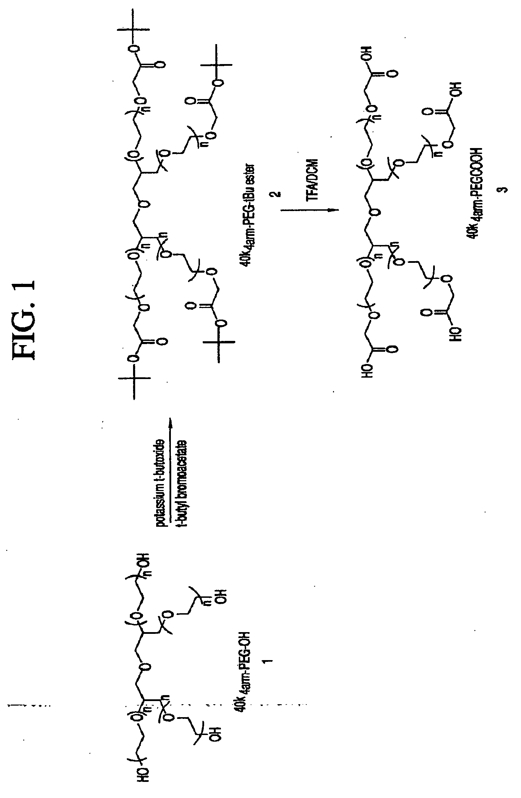

FIG. 1 schematically illustrates a reaction scheme of preparing four-arm polyethylene glycol acids, as described in Examples 1-2.

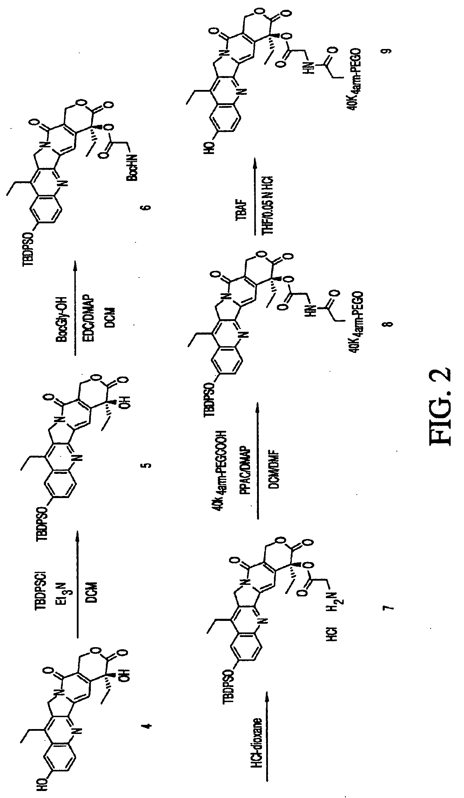

FIG. 2 schematically illustrates a reaction scheme of preparing 4arm-PEG-Gly-(7-ethyl-10-hydroxycamptothecin), as described in Examples 3-7.

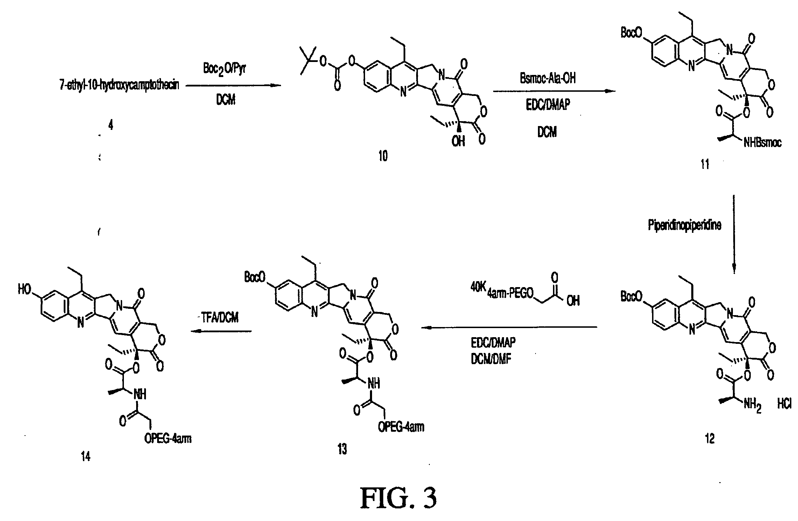

FIG. 3 schematically illustrates a reaction scheme of preparing 4arm-PEG-Ala-(7-ethyl-10-hydroxycamptothecin), as described in Examples 8-12.

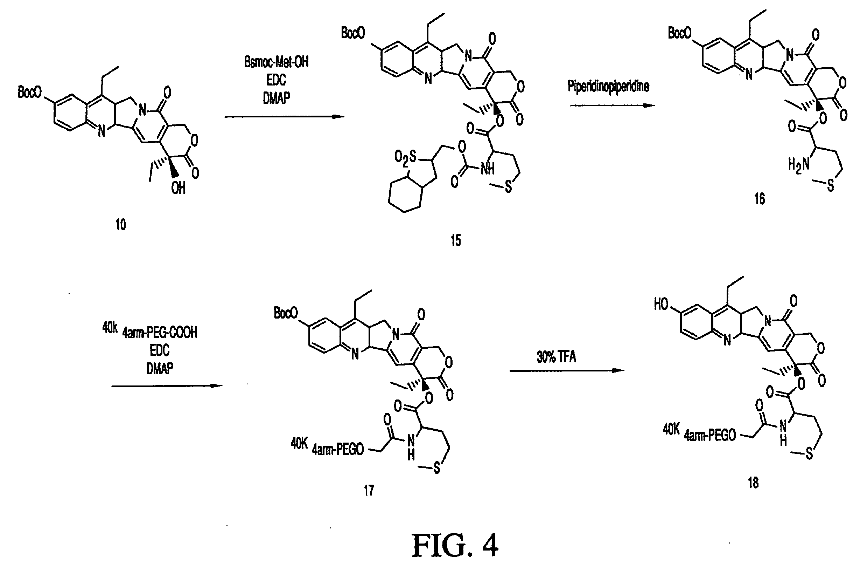

FIG. 4 schematically illustrates a reaction scheme of preparing 4arm-PEG-Met-(7-ethyl-10-hydroxycamptothecin), as described in Examples 13-16.

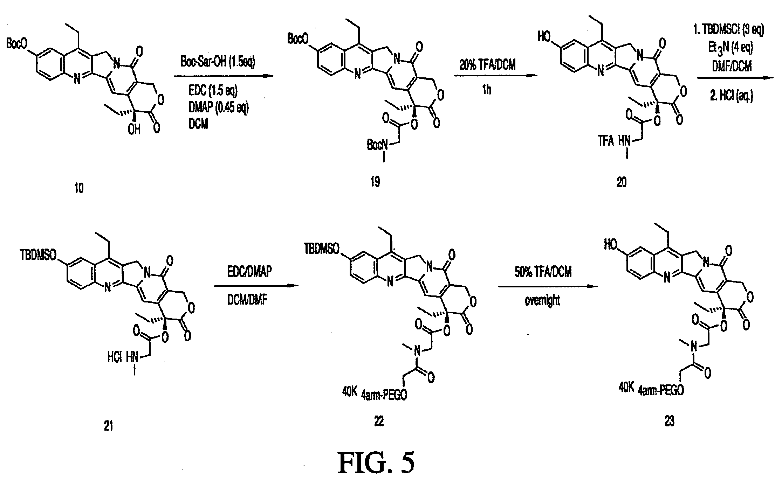

FIG. 5 schematically illustrates a reaction scheme of preparing 4arm-PEG-Sar-(7-ethyl-10-hydroxycamptothecin) described in Examples 17-21.
FIG. 6 shows stability of 4arm-PEG-Gly-(7-ethyl-10-hydroxycamptothecin), as described in Example 24.
FIG. 7 shows effect of pH on stability of 4arm-PEG-Gly-(7-ethyl-10-hydroxycamptothecin), as described in Example 24.
FIGs. 8A and 8B show pharmacokinetic profiles of 4arm-PEG-Gly-(7-ethyl-10-hydroxy-camptothecin), as described in Example 25.
FIGs. 9A and 9B show anticancer effects on survival rate in pseudometastatic human neuroblastoma(HTLA-230) xenografted mice, as described in Example 26.
FIG. 10 shows anticancer effects on survival rate in orthotopic human neuroblastoma (GI-LI-N) xenografted mice, as described in Example 27.
FIG. 11 shows tumor regression in orthotopic human neuroblastoma (GI-LI-N) xenografted mice, as described in Example 28.
FIG. 12A shows immunohistochemical studies of tumor makers as described in Example 29, based on Example 27. Tumor sections were immunostained for NB84 as a neuroblastoma cell marker, and Ki-67 as a tumor cell proliferation marker.
FIG. 12B shows quantitative measurements of NB84 and Ki-67 positive cells in mice treated with control, CPT-11 and compound 9, respectively.
FIG. 13 shows survival of mice treated with compound 9 compared to CPT-11 at MTD in GI-LI-N xenografted mice, as described in Example 30.
FIG. 14A shows antitumor efficacy of compound 9 in mice xenografted with human neuroblastoma cells NXS2 at 5x10⁴ cells, as described in Example 31.
FIG. 14B shows antitumor efficacy of compound 9 in mice xenografted with human neuroblastoma cells NXS2 at 5x10⁵ cells, as described in Example 31.
FIG. 15A shows the time-dependent antitumor effects in SH-SY5Y xenografted mice, comparing compound 9 to CAMPTOSAR (CPT-11 in pharmaceutical formulation) based on the bioluminescence measured from xenografted luciferase-expressing neuroblastoma cells.
FIG. 15B illustrates micrographs of SH-SY5Y xenografted mice, comparing the bioluminescence intensity measured from xenografted luciferase-expressing neuroblastoma cells. Although the original micrographs were in color (color not reproduced herein), the intensity of the luminescence in this figure, as a correlation of tumor burden, is readily apparent.

### DETAILED DESCRIPTION OF THE INVENTION

### A. OVERVIEW

The present invention relates to compounds of formula (I) below for use in methods of treating neuroblastoma in a mammal. The methods include administering an effective amount of a compound of Formula (I) or a pharmaceutically acceptable salt thereof to a mammal in need thereof. In one aspect, the compounds of Formula (I) have the structure: 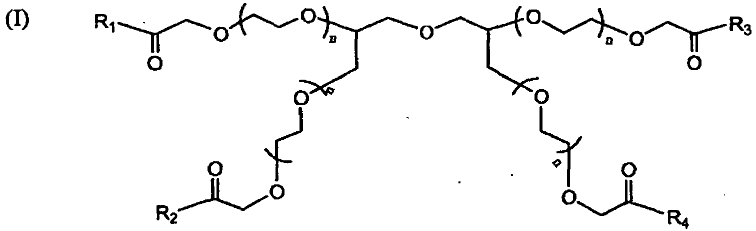 wherein
R₁, R₂, R₃ and R₄ are independently OH or 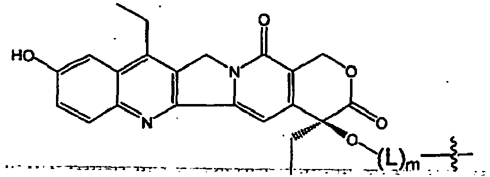 wherein
L is a bifunctional linker, and each L is the same or different when (m) is equal to or greater than 2;
(m) is a positive integer such as, for example, from about 1 to about 10
(for example, 1, 2, 3, 4, 5 or 6), and preferably 1; and
(n) is a positive integer, preferably from about 28 to about 341, more preferably from about 114 to about 239, yet more preferably about 227; provided that R₁, R₂, R₃ and R₄ are not all OH.

In one preferred embodiment, the method includes a compound of Formula (I) as part of a pharmaceutical composition, and R₁, R₂, R₃ and R₄ are all:

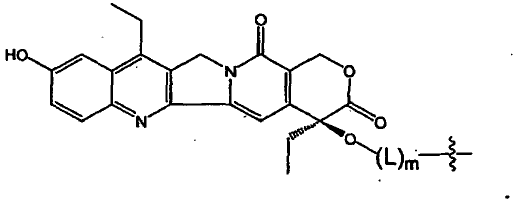

In more preferred aspect, the treatment includes administering a compound having the structure: 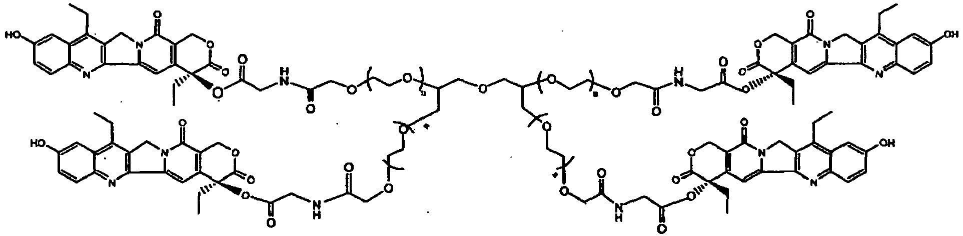 wherein (n) is about 227 so that the polymeric portion of the compound has a total number average molecular weight of about 40,000 daltons.

### B. COMPOUND OF FORMULA (I):

### 1. MULTI-ARM POLYMERS

The polymeric portion of the compounds described herein includes multi-arm PEG's attached to 20-OH group of 7-ethyl-10-hydroxycamptothecin. In one aspect of the present invention; the polymeric prodrugs of 7-ethyl-10-hydroxycamptothecin include four-arm PEG, prior to conjugation, having the following structure of 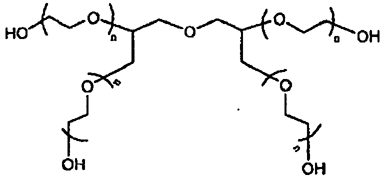 wherein (n) is a positive integer.

The multi-arm PEG's are those described in NOF Corp. Drug Delivery System catalog, Ver. 8, April 2006.

In one preferred embodiment of the invention, the degree of polymerization for the polymer (n) is from about 28 to about 341 to provide polymers having a total number average molecular weight of from about 5,000 Da to about 60,000 Da, and preferably from about 114 to about 239 to provide polymers having a total number average molecular weight of from about 20,000 Da to about 42,000 Da. (n) represents the number of repeating units in the polymer chain and is dependent on the molecular weight of the polymer. In one particularly preferred embodiment of the invention, (n) is about 227 to provide the polymeric portion having a total number average molecular weight of about 40,000 Da.

### 2. BIFUNCTIONAL LINKERS

In certain preferred aspects of the present invention, bifunctional linkers include an amino acid. The amino acid which can be selected from any of the known naturally-occurring L-amino acids is, e.g., alanine, valine, leucine, isoleucine, glycine, serine, threonine, methionine, cysteine, phenylalanine, tyrosine, tryptophan, aspartic acid, glutamic acid, lysine, arginine, histidine, proline, and/or a combination thereof, to name but a few. In alternative aspects, L can be a peptide residue. The peptide can range in size, for instance, from about 2 to about 10 amino acid residues (e.g., 2, 3, 4, 5, or 6).

Derivatives and analogs of the naturally occurring amino acids, as well as various art-known non-naturally occurring amino acids (D or L), hydrophobic or non-hydrophobic, are also contemplated to be within the scope of the invention. Simply by way of example, amino acid analogs and derivates include:
2-aminoadipic acid, 3-aminoadipic acid, beta-alanine, beta-aminopropionic acid,
2-aminobutyric acid, 4-aminobutyric acid, piperidinic acid, 6-aminocaproic acid,
2-aminoheptanoic acid, 2-aminoisobutyric acid, 3-aminoisobutyric acid,
2-aminopimelic acid, 2,4-aminobutyric acid, desmosine, 2,2-diaminopimelic acid,
2,3-diaminopropionic acid, N-ethylglycine, N-ethylasparagine, 3-hydroxyproline,
4-hydroxyproline, isodesmosine, allo-isoleucine, N-methylglycine or sarcosine,
N-methyl-isoleucine, 6-N-methyllysine, N-methylvaline, norvaline, norleucine, ornithine,
and others too numerous to mention, that are listed in 63 Fed. Reg., 29620, 29622.
Some preferred L groups include glycine, alanine, methionine or sarcosine. For example, the compounds can be among: 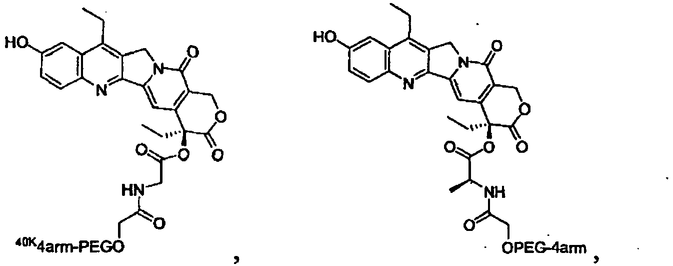 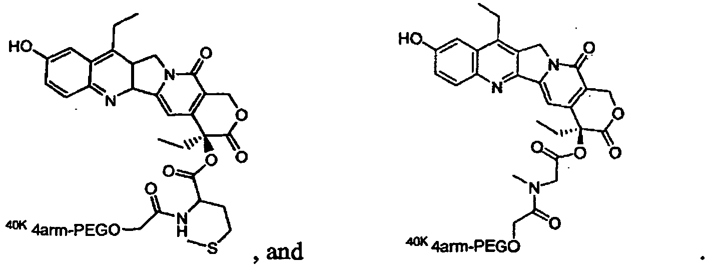 For ease of the description and not limitation, only one arm of the four-arm PEG is shown. One arm, up to four arms of the four-arm PEG can be conjugated with 7-ethyl-10-hydroxy-camptothecin.

More preferably, the treatment described herein employs compounds including a glycine as the linker group (L).

In an alternative aspect of the present invention, L after attachment between the polymer and 7-ethyl-10-hydroxycamptothecin can be selected among:
-[C(=O)]ᵥ(CR₂₂R₂₃)ₜ-,
-[C(=O)]ᵥ(CR₂₂R₂₃)ₜ-O-,
-[C(=O)]ᵥ(CR₂₂R₂₃)ₜ-NR₂₆-,
-[C(=O)]ᵥO(CR₂₂R₂₃)ₜ-,
-[C(=O)]ᵥO(CR₂₂R₂₃)ₜO-,
-[C(=O)]ᵥO(CR₂₂R₂₃)ₜNR₂₆-,
-[C(=O)]ᵥNR₂₁(CR₂₂R₂₃)ₜ-,
-[C(=O)]ᵥNR₂₁(CR₂₂R₂₃)ₜO-,
-[C(=O)]ᵥNR₂₁(CR₂₂R₂₃)ₜNR₂₆-,
-[C(=O)]ᵥ(CR₂₂R₂₃O)ₜ-,
-[C(=O)]ᵥO(CR₂₂R₂₃O)ₜ-,
-[C(=O)]ᵥNR₂₁(CR₂₂R₂₃O)ₜ-,
-[C(=O)]ᵥ(CR₂₂R₂₃O)ₜ(CR₂₄R₂₅)_{y}-,
-[C(=O)]ᵥO(CR₂₂R₂₃O)ₜ(CR₂₄R₂₅)_{y}-,
-[C(=O)]ᵥNR₂₁(CR₂₂R₂₃O)ₜ(CR₂₄R₂₅)_{y}-,
-[C(=O)]ᵥCR₂₂R₂₃O)ₜ(CR₂₄R₂₅)_{y}O-,
-[C(=O)]ᵥ(CR₂₂R₂₃)ₜ(CR₂₄R₂₅)_{y}-,
-[C(=O)]ᵥO(CR₂₂R₂₃O)ₜ(CR₂₄R₂₅)_{y}O-,
-[C(=O)]ᵥO(CR₂₂R₂₃)ₜ(CR₂₄R₂₅O)_{y}-,
-[C(=C)]ᵥNR₂₁(CR₂₂R₂₃O)ₜ(CR₂₄R₂₅)_{y}O-,
-[C(=O)]ᵥNR₂₁(CR₂₂R₂₃)ₜ(CR₂₄R₂₅O)_{y}-,
-[C(=O)]ᵥ(CR₂₂R₂₃)ₜO-(CR₂₈R₂₉)_{t'}-,
-[C(=O)]ᵥCR₂₂R₂₃)ₜNR₂₆-(CR₂₈R₂₉)_{t'}-,
-[C(=O)]ᵥ(CR₂₂R₂₃)ₜS-(CR₂₈R₂₉)_{t'}-,
-[C(=O)]ᵥO(CR₂₂R₂₃)ₜ-(CR₂₈R₂₉)_{t'}-,
-[C(=O)]ᵥO(CR₂₂R₂₃)ₜNR₂₆-(CR₂₈R₂₉)_{t'}-,
-[C(=O)]ᵥO(CR₂₂R₂₃)ₜS-(CR₂₈R₂₉)_{t'}-,
-[C(=O)]ᵥNR₂₁(CR₂₂R₂₃)ₜO-(CR₂₈R₂₉)_{t'}-,
-[C(=C)]ᵥNR₂₁(CR₂₂R₂₃)ₜNR₂₆-(CR₂₈R₂₉)_{t'}-,
-[C(=O)]ᵥNR₂₁(CR₂₂R₂₃)ₜS-(CR₂₈R₂₉)_{t'}-,
-[C(=O)]ᵥ(CR₂₂R₂₃CR₂₈R₂₉O)ₜNR₂₆-,
-[C(=O)]ᵥ(CR₂₂R₂₃CR₂₈R₂₉O)ₜ-,
-[C(=O)]ᵥO(CR₂₂R₂₃CR₂₈R₂₉O)ₜNR₂₆-,
-[C(=O)]ᵥ(CR₂₂R₂₃CR₂₈R₂₉O)ₜ-,
-[C(=O)]NR₂₁CR₂₂R₂₃CR₂₈R₂₉O)ₜNR₂₆-,
-[C(=O)]ᵥNR₂₁(R₂₂R₂₃CR₂₈R₂₉O)ₜ-,
-[C(=O)]ᵥ(CR₂₂R₂₃CR₂₈R₂₉O)ₜ(CR₂₄R₂₅)_{y}-,
-[C(=O)]ᵥO(CR₂₂R₂₃CR₂₈R₂₉O)ₜ(CR₂₄R₂₅)_{y}-,
-[C(=O)]ᵥNR₂₁(CR₂₂R₂₃CR₂₈R₂₉O)ₜ(CR₂₄R₂₅)_{y}-,
-[C(=O)]ᵥNR₂₁(CR₂₂R₂₃CR₂₈R₂₉O)ₜ(CR₂₄R₂₅)_{y}-,
-[C(=O)]ᵥ(CR₂₂R₂₃CR₂₈R₂₉O)ₜ(CR₂₄R₂₅)_{y}O-,
-[C(=O)]ᵥ(CR₂₂R₂₃)ₜ(CR₂₄R₂₅CR₂₈R₂₉O)_{y}-,
-[C(=O)]ᵥ(CR₂₂R₂₃)ₜ(CR₂₄R₂₅CR₂₈R₂₉O)_{y}NR₂₆-,
-[C(=O)]ᵥO(CR₂₂R₂₃CR₂₈R₂₉O)ₜ(CR₂₄R₂₅)_{y}O-,
-[C(=O)]ᵥO(CR₂₂R₂₃)ₜ(CR₂₄R₂₅CR₂₈R₂₉O)_{y}-,
-[C(=O)]ᵥO(CR₂₂R₂₃)ₜ(CR₂₄CR₂₅CR₂₈R₂₉O)_{y}NR₂₆-,
-[C(=O)]ᵥNR₂₁(CR₂₂R₂₃CR₂₈R₂₉O)ₜ(CR₂₄R₂₅)_{y}O-,
-[C(=O)]ᵥNR₂₁(CR₂₂R₂₃)ₜ(CR₂₄R₂₅CR₂₈R₂₉O)_{y}-,
-[C(=O)]ᵥNR₂₁(CR₂₂R₂₃)ₜ(CR₂₄R₂₅CR₂₈R₂₉O)_{y}NR₂₆-, 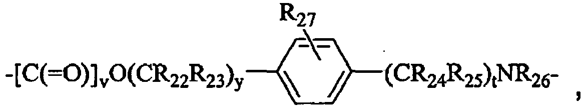 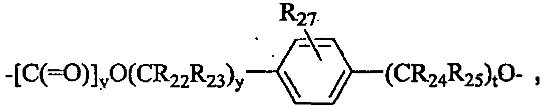 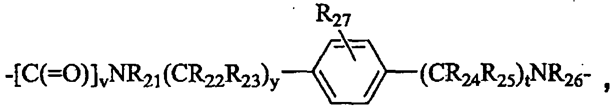 and 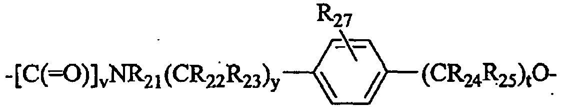 wherein:
   R₂₁-R₂₉ are independently selected among hydrogen, amino, substituted amino, azido, carboxy, cyano, halo, hydroxyl, nitro, silyl ether, sulfonyl, mercapto, C₁₋₆ alkylmercapto, arylmercapto, substituted arylmercapto, substituted C₁₋₆ alkylthio, C₁₋₆alkyls, C₂₋₆ alkenyl, C₂₋₆ alkynyl, C₃₋₁₉ branched alkyl, C₃₋₈ cycloalkyl, C₁₋₆ substituted alkyl, C₂₋₆ substituted alkenyl, C₂₋₆ substituted alkynyl, C₃₋₈ substituted cycloalkyl, aryl, substituted aryl, heteroaryl, substituted heteroaryl, C₁₋₆ heteroalkyl, substituted C₁₋₆heteroalkyl, C₁₋₆ alkoxy, aryloxy, C₁₋₆heteroalkoxy, heteroaryloxy, C₂₋₆ alkanoyl, arylcarbonyl, C₂₋₆ alkoxycarbonyl, aryloxycarbonyl, C₂₋₆ alkanoyloxy, arylcarbonyloxy, C₂₋₆ substituted alkanoyl, substituted arylcarbonyl, C₂₋₆ substituted alkanoyloxy, substituted aryloxycarbonyl, C₂₋₆ substituted alkanoyloxy, substituted and arylcarbonyloxy;
   (t), (t') and (y) are independently chosen from zero or a positive integer, preferably from about 1 to about 10 such as 1, 2, 3, 4, 5 and 6; and
   (v) is 0 or 1.

In some preferred embodiments, L can include:
- [C(=O)]ᵥ(CH₂)ₜ-,
- [C(=O)]ᵥCH₂)ₜ-O-,
- [C(-O)]ᵥ(CH₂)ₜ-NR₂₆-,
- [C(=O)]ᵥO(CH₂)ₜ-,
- [C(₌O)]ᵥO(CH₂)ₜO-,
- [C(=O)]ᵥO(CH₂)ₜNH-,
- [C(=O)]ᵥNH(CH₂)ₜ-,
- [C(=O)]ᵥNH(CH₂)ₜO-,
- [C(=O)]ᵥNH(CH₂)ₜNH-,
- [C(=O)]ᵥ(CH₂O)ₜ-,
- [C(=O)]ᵥO(CH₂O)ₜ-,
- [C(=O)]ᵥNH(CH₂O)ₜ-,
- [C(=O)]ᵥ(CH₂O)ₜ(CH₂)_{y}-,
- [C(=O)]ᵥO(CH₂O)ₜH₂)_{y}-,
- [C(=O)]ᵥNH(CH₂O)ₜ(CH₂₅)_{y}-,
- [C(=O)]ᵥ(CH₂O)ₜ(CH₂)_{y}O-,
- [C(=O)]ᵥ(CH₂)ₜ(CH₂O)_{y}-,
- [C(=O)]ᵥO(CH₂O)ₜ(CH₂O)ₜ(CH₂)_{y}O-,
- [C(=O)]ᵥO(CH₂)ₜ(CH₂O)_{y}-,
- [C{=O)]ᵥNH(CH₂O)ₜ(CH₂)_{y}O-,
- [C(=O)]ᵥNH(CR₂₂R₂₃)ₜ(CH₂O)_{y}-,
- [C(=O)]ᵥ(CH₂)ₜO-(CH₂)_{t'}-,
- [C(=O)]ᵥ(CH₂)ₜNH-(CH₂)_{t'}-,
- [C(=O)]ᵥ(CH₂)ₜS-(CH₂)_{t'}-,
- [C(=O)]ᵥO(CH₂)ₜO-(CH₂)_{t'}-,
- [C(=O)]ᵥO(CH₂)ₜNH-(CH₂)_{t'}-,
- [C(=O)]ᵥO(CH₂)ₜS-(CH₂)_{t'}-,
- [C(=O)]ᵥNH(CR₂₂R₂₃)ₜO-(CH₂)_{t'}-,
- [C(=O)]ᵥNH(CH₂)ₜNH-(CH₂)_{t'}-,
- [C(=O)]ᵥNH(CH₂)ₜS-(CH₂)_{t'},
- [C(=O)]ᵥ(CH₂CH₂O)ₜNR₂₆-,
- [C(=O)]ᵥ(CH₂CH₂O)ₜ-,
- [C(=O)]ᵥO(CH₂CH₂O)ₜNH-,
- [C(=O)]ᵥO(CH₂CH₂O)ₜ-,
- [C(=O)]ᵥNH(CH₂CH₂O)ₜNH-,
- [C(=O)]ᵥ(CH₂CH₂O)ₜ-,
- [C(=O)]ᵥ(CH₂CH₂O)ₜ(CH₂)_{y}-,
- [C(=O)]ᵥO(CH₂CH₂O)ₜ(CH₂)_{y}-,
- [C(=O)]ᵥNH(CH₂CH₂O)ₜ(CH₂)_{y}-,
- [C(=O)]ᵥ(CH₂CH₂O)ₜ(CH₂)yO-,
- [C(=O)]ᵥ(CH₂)ₜ(CH₂CH₂O)_{y}-,
- [C(=O)]ᵥ(CH₂)ₜCH₂CH₂O)_{y}NH-,
- [C(=O)]ᵥO(CH₂CH₂O)ₜ(CH₂)_{y}O-,
- [C(=O)]ᵥO(CH₂)ₜ(CH₂CH₂O)_{y}-,
- [C(=O)]ᵥO(CH₂)ₜ(CH₂CH₂O)_{y}NH-,
- [C(=O)]ᵥNH(CH₂CH₂O)ₜ(CH₂)_{y}O-,
- [C(=O)]ᵥNH(CH₂)ₜ(CH₂CH₂O)_{y}-,
- [C(=O)]ᵥNH(CH₂)ₜ(CH₂CH₂O)_{y}NH-, 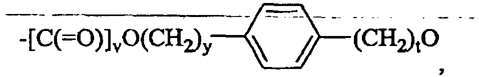 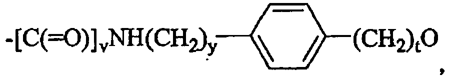 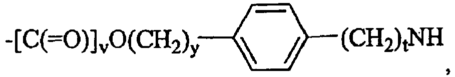 and 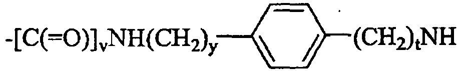 wherein (t), (t') and (y) are independently chosen from zero or a positive integer, preferably from about 1 to about 10 (e.g., 1, 2, 3, 4, 5, and 6); and
   (v) is 0 or 1.

In some aspects of the present invention, the compounds of Formula (I) include from 1 to about 10 units (e.g., 1, 2, 3, 4, 5, or 6) of the bifunctional linker. In some preferred aspects of the present invention, the compounds include one unit of the bifunctional linker and thus (m) is 1.

Additional linkers are found in Table 1 of Greenwald et al. (Bioorganic & Medicinal Chemistry, 1998, 6:551-562).

### 3. SYNTHESIS OF PRODRUGS

Generally, the polymeric prodrugs employed in treatment are prepared by reacting one or more equivalents of an activated multi-arm polymer with, for example, one or more equivalents per active site of amino acid-(20)-7-ethyl-10-hydroxycamptothecin under conditions which are sufficient to effectively cause the amino group to undergo a reaction with the carboxylic acid of the polymer and form a linkage. Details of the synthesis are described in US Patent Publication No. 2007/0197575.

More specifically, the methods can include:
1) providing one equivalent of 7-ethyl-10-hydroxycamptothecin containing an available 20-hydroxyl group and one or more equivalents of a bifunctinal linker containing an available carboxylic acid group;
2) reacting the two reactants to form a 7-ethyl-10-hydroxycamptothecin-bifunctional linker intermediate in an inert solvent such as dichloromethane (DCM) (or dimethylformamide (DMF), chloroform, toluene or mixtures thereof) in the presence of a coupling reagent such as 1,(3-dimethyl aminopropyl) 3-ethyl carbodiimide EDC), (or 1,3-diisopropylcarbodiimide (DIPC), any suitable dialkyl carbodiimide, Mukaiyama reagents, (e.g. 2-halo-1-alkyl-pyridinium halides) or propane phosphonic acid cyclic anhydride (PPACA), etc) and a suitable base such as 4-dimethylaminopyridine (DMAP); and
3) reacting one or more equivalents per active site (fore example, 2 equivalents in Example) of the resulting intermediate having an amine group and one equivalent of an activated polymer, such as a PEG-acid in an inert solvent such as dichloromethane (DCM) (or dimethylformamide (DMF), chloroform, toluene or mixtures thereof) in the presence of a coupling reagent such as 1,(3-dimethyl aminopropyl) 3-ethyl carbodiimide (EDC), PPAC (or 1,3-diisopropylcarbodiimide (DIPC), any suitable dialkyl carbodiimide, Mukaiyama reagents, (e.g. 2-halo-1-alkyl-pyridinium halides) or propane phosphonic acid cyclic anhydride (PPACA), etc.), and a suitable base such as 4-dimethylaminopyridine (DMAP), which are available, for example, from commercial sources such as Sigma Chemical, or synthesized using known techniques, at a temperature from 0EC up to 22EC.

In one preferred aspect, the 10-hydroxyl group of 7-ethyl-10-hydroxycamptothecin is protected prior to step 1).

Protection of the aromatic OH of 10-hydroxyl group in 7-ethyl-10-hydroxycamptothecin are preferred because the protected 7-ethyl-10-hydroxycamptothecin intermediates thereof have better solubility and can be purified in highly pure form efficiently and effectively. For example, silyl-containing protecting groups such as TBDPSCl (*t*-butyldiphenylsilyl chloride), TBDMSCl (t-butyldimethylsilyl chloride) and TMSCl (trimethylsilyl chloride) can be used to protect the 10-hydroxyl group in 7-ethyl-10-hydroxycamptothecin.

The activated polymer, i.e., a polymer containing 1-4 terminal carboxyl acid groups can be prepared, for example, by converting NOF Sunbright-type having terminal OH groups into the corresponding carboxyl acid derivatives using standard techniques well known to those of ordinary skill. See, for example, Examples 1-2 herein as well as commonly assigned U.S. Patent No. 5,605,976 and U.S. Patent Publication No. 2007/0173615.

The first and second coupling agents can be the same or different.

Examples of preferred bifunctional linker groups include glycine, alanine, methionine, sarcosine, etc. and syntheses are described in the Examples.

According to the present invention, the compounds administered include: 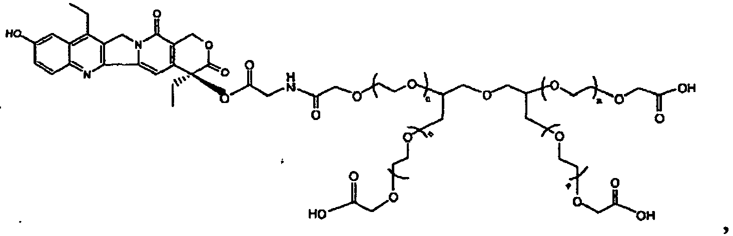 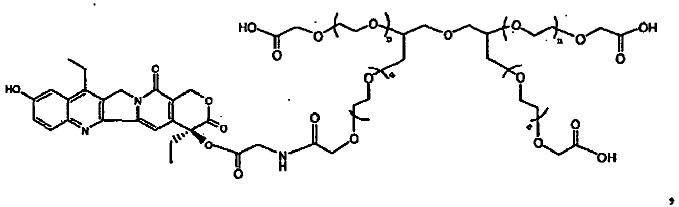 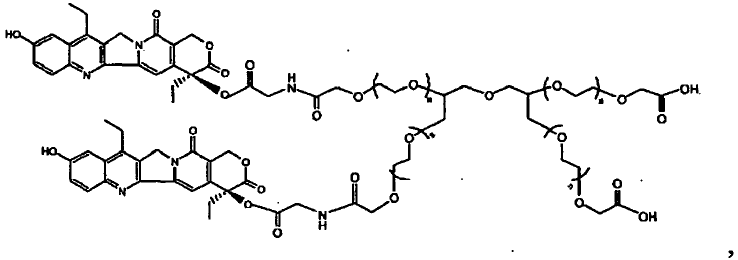 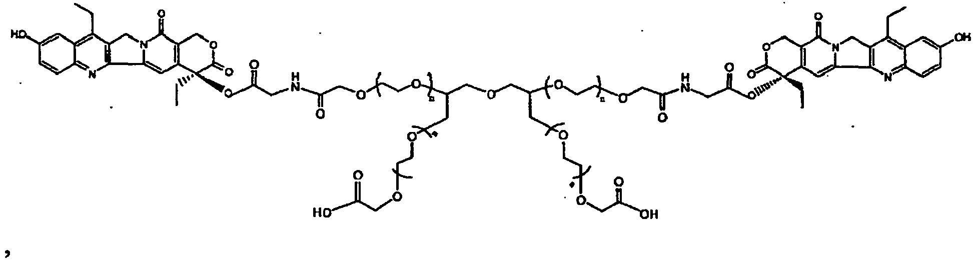 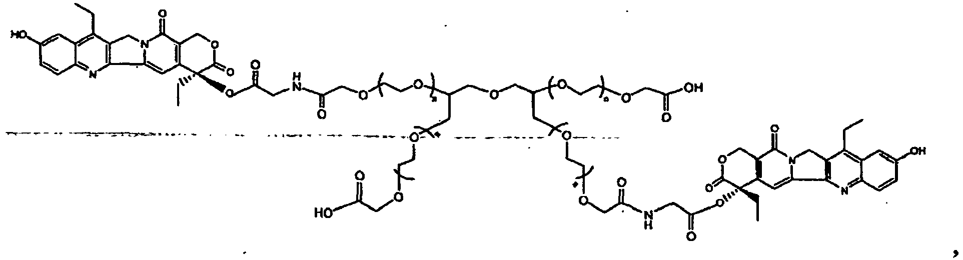 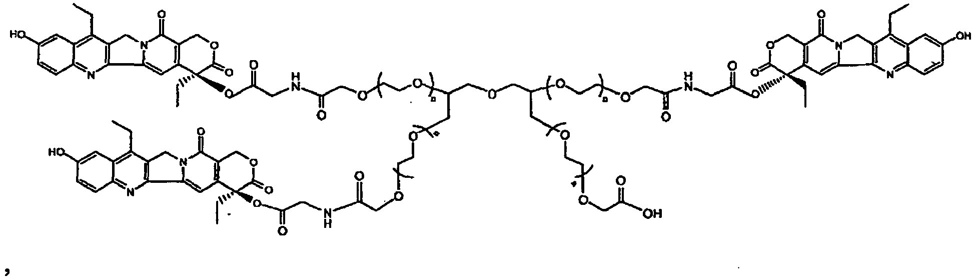 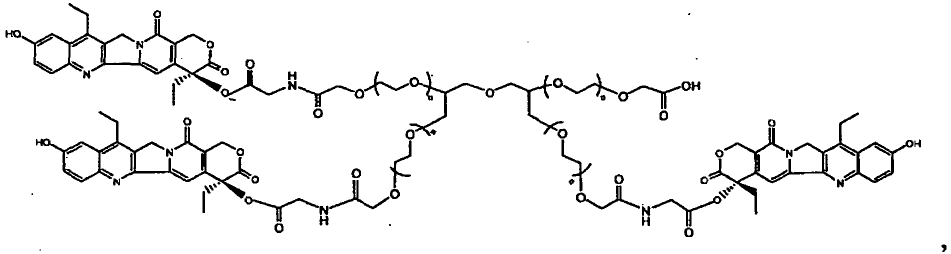 and

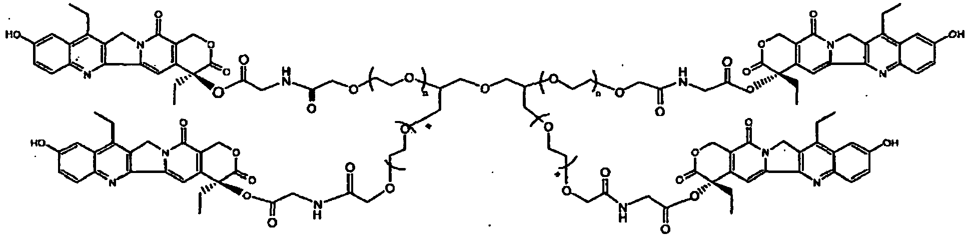

One particularly preferred embodiment includes administering a compound having the structure 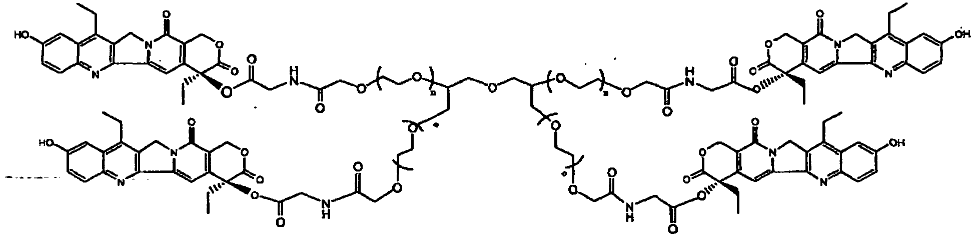 wherein all four arms of the polymer are conjugated to 7-ethyl-10-hydroxycamptothecin through glycine and the polymer portion has a total molecular weight of about 40,000 daltons.

### C. COMPOSITIONS/FORMULATIONS

Pharmaceutical compositions containing the polymer conjugates of the present invention may be manufactured by processes well known in the art, e.g., using a variety of well-known mixing, dissolving, granulating, levigating, emulsifying, encapsulating, entrapping or lyophilizing processes. The compositions may be formulated in conjunction with one or more physiologically acceptable carriers comprising excipients and auxiliaries which facilitate processing of the active compounds into preparations which can be used pharmaceutically. Proper formulation is dependent upon the route of administration chosen. Parenteral routes are preferred in many aspects of the invention.

For injection, including, without limitation, intravenous, intramusclular and subcutaneous injection, the compounds of the invention may be formulated in aqueous solutions, preferably in physiologically compatible buffers such as physiological saline buffer or polar solvents including, without limitation, a pyrrolidone or dimethylsulfoxide.

The compounds described herein may also be formulated for parenteral administration, e.g., by bolus injection or continuous infusion. Formulations for injection may be presented in unit dosage form, e.g., in ampoules or in multi-dose containers. Useful compositions include, without limitation, suspensions, solutions or emulsions in oily or aqueous vehicles, and may contain adjuncts such as suspending, stabilizing and/or dispersing agents. Pharmaceutical compositions for parenteral administration include aqueous solutions of a water soluble form, such as, without limitation, a salt (preferred) of the active compound. Additionally, suspensions of the active compounds may be prepared in a lipophilic vehicle. Suitable lipophilic vehicles include fatty oils such as sesame oil, synthetic fatty acid esters such as ethyl oleate and triglycerides, or materials such as liposomes. Aqueous injection suspensions may contain substances that increase the viscosity of the suspension, such as sodium carboxymethyl cellulose, sorbitol, or dextran. Optionally, the suspension may also contain suitable stabilizers and/or agents that increase the solubility of the compounds to allow for the preparation of highly concentrated solutions. Alternatively, the active ingredient may be in powder form for constitution with a suitable vehicle, e.g., sterile, pyrogen-free water, before use.

For oral administration, the compounds can be formulated by combining the active compounds with pharmaceutically acceptable carriers well-known in the art. Such carriers enable the compounds of the invention to be formulated as tablets, pills, lozenges, dragees, capsules, liquids, gels, syrups, pastes, slurries, solutions, suspensions, concentrated solutions and suspensions for diluting in the drinking water of a patient, premixes for dilution in the feed of a patient, and the like, for oral ingestion by a patient. Pharmaceutical preparations for oral use can be made using a solid excipient, optionally grinding the resulting mixture, and processing the mixture of granules, after adding other suitable auxiliaries if desired, to obtain tablets or dragee cores. Useful excipients are, in particular, fillers such as sugars, including lactose, sucrose, mannitol, or sorbitol, cellulose preparations such as, for example, maize starch, wheat starch, rice starch and potato starch and other materials such as gelatin, gum tragacanth, methyl cellulose, hydroxypropyl- methylcellulose, sodium carboxymethylcellulose, and/or polyvinylpyrrolidone (PVP). If desired, disintegrating agents may be added, such as cross-linked polyvinyl pyrrolidone, agar, or alginic acid. A salt such as sodium alginate may also be used.

For administration by inhalation, the compounds of the present invention can conveniently be delivered in the form of an aerosol spray using a pressurized pack or a nebulizer and a suitable propellant.

The compounds may also be formulated in rectal compositions such as suppositories or retention enemas, using, e.g., conventional suppository bases such as cocoa butter or other glycerides.

In addition to the formulations described previously, the compounds may also be formulated as depot preparations. Such long acting formulations may be administered by implantation (for example, subcutaneously or intramuscularly) or by intramuscular injection. A compound of this invention may be formulated for this route of administration with suitable polymeric or hydrophobic materials (for instance, in an emulsion with a pharmacologically acceptable oil), with ion exchange resins, or as a sparingly soluble derivative such as, without limitation, a sparingly soluble salt.

Other delivery systems such as liposomes and emulsions can also be used.

Additionally, the compounds may be delivered using a sustained-release system, such as semi-permeable matrices of solid hydrophobic polymers containing the therapeutic agent Various sustained-release materials have been established and are well known by those skilled in the art. Sustained-release capsules may, depending on their chemical nature, release the compounds for a few weeks up to over 100 days. Depending on the chemical nature and the biological stability of the particular compound, additional stabilization strategies may be employed.

### D. DOSAGES

A therapeutically effective amount refers to an amount of a compound effective to prevent, alleviate or ameliorate a 7-ethyl-10-hydroxycamptothecin-susceptible condition. Determination of a therapeutically effective amount is well within the capability of those skilled in the art, especially in light of the disclosure herein.

For any compound used in the methods of the present invention, the therapeutically effective amount can be estimated initially from *in vitro* assays. Then, the dosage can be formulated for use in animal models so as to achieve a circulating concentration range that includes the effective dosage. Such information can then be used to more accurately determine dosages useful in patients.

The amount of the composition, e.g., used as a prodrug, that is administered will depend upon the parent molecule included therein (in this case, 7-ethyl-10-hydroxycamptothecin). Generally, the amount of prodrug used in the treatment methods is that amount which effectively achieves the desired therapeutic result in mammals. Naturally, the dosages of the various prodrug compounds can vary somewhat depending upon the parent compound, rate of *in vivo* hydrolysis, molecular weight of the polymer, etc. In addition, the dosage, of course, can vary depending upon the dosage form and route of administration.

In general, however, the polymeric ester derivatives of 7-ethyl-10-hydroxy camptothecin described herein can be administered in amounts ranging from about 0.3 to about 90 mg/m² body surface, and preferably from about 0.5 to about 50 mg/ m² body surface/dose, yet preferably from about 1 to about 18 mg/ m² body surface/dose, and even more preferably from about 1.25 mg/m² body surface/dose to about 16.5 mg/m² body surface/dose for systemic delivery.

The compounds can be administered in amounts ranging from about 0.3 to about 90 mg/ m² body surface/week such as, for example, from about 1 to about 18 mg/ m² body surface/week. In particular embodiments, the dose regimens can be, for example, from 5-7 mg/m² body surface weekly for 3 weeks in 4-week cycles, from 1.25-45 mg/m² one injection every 3 weeks, and/or from 1-16 mg/m² three injections weekly in a four week cycle.

Preferably, the amounts of the compounds described herein range from about 1 to about 18 mg/m² body surface/dose. More preferably, the amounts administered can range from about 1.25 to about 16.5 mg/m² body surface/dose. Some preferred doses include one of the following: 1.25, 2.5, 5, 10, and 16.5 mg/m²/dose. One embodiment includes 5 mg/m² body surface/dose.

The treatment protocol can be based on a single dose administered once every three weeks or divided into multiple doses which are given as part of a multi-week treatment protocol. Thus, the treatment regimens can include one dose every three weeks for each treatment cycle and, alternatively one dose weekly for three weeks followed by one week off for each cycle. It is also contemplated that the treatment will be given for one or more cycles until the desired clinical result is obtained.

For purposes of the present invention, the weight given above represents the weight of 7-ethyl-10-hydroxycamptothecin present in the PEG-conjugated 7-ethyl-10-hydroxycamptothecin employed for treatment. The actual weight of the PEG-conjugated 7-ethyl-10-hydroxycamptothecin will vary depending on the loading of the PEG (e.g., optionally from one to four moles of 7-ethyl-10-hydroxycamptothecin per mole of PEG.).

The range set forth above is illustrative and those skilled in the art will determine the optimal dosing of the prodrug selected based on clinical experience and the treatment indication. Moreover, the exact formulation, route of administration and dosage can be selected by the individual physician in view of the patient's condition. The precise dose will depend on the stage and severity of the condition, and the individual characteristics of the patient being treated, as will be appreciated by one of ordinary skill in the art.

Additionally, toxicity and therapeutic efficacy of the compounds described herein can be determined by standard pharmaceutical procedures in cell cultures or experimental animals using methods well-known in the art.

In some preferred embodiments, the treatment protocol includes administering the amount ranging from about 1.25 to about 16.5 mg/m² body surface/dose weekly for three weeks, followed by one week without treatment and repeating for about 3 cycles or more until the desired results are observed. The amount administered per each cycle can range more preferably from about 2.5 to about 16.5 mg/m² body surface/dose.

In one particular embodiment, the polymeric ester derivatives of 7-ethyl-10-hydroxycamptothecin can be administered one dose such as 5 or 10 mg/m² weekly for three weeks, followed by one week without treatment. The dosage of treatment cycle can be designed as an escalating dose regimen when two or more treatment cycles are applied. The polymeric drug is preferably administered via IV infusion.

Alternative embodiments include: for the treatment of pediatric patients, a regimen based on a protocol of about 1.85 mg/m² body surface/dose daily for 5 days every three weeks, a protocol of from about 1.85 to about 7.5 mg/m² body surface/dose daily for 3 days every 25 days, or a protocol of about 22.5 mg/m² body surface/dose once every three weeks, and for the treatment of adult patients, a protocol based on about 13 mg/m² body surface/dose every three weeks or about 4.5 mg/m² body surface/dose weekly for four weeks every six weeks. The compounds described herein can be administered in combination with one or more anticancer agents. In one embodiment, the combination therapy includes a protocol of about 0.75 mg/m² body surface/dose daily for 5 days each cycle in combination with a second agent.

Alternatively, the compounds administered can be based on body weight. The dosage range for systemic delivery of a compound of Formula (I) in a mammal will be from about 1 to about 100 mg/kg/week and is preferably from about 2 to about 60 mg/kg/week. Thus, the amounts can range from about 0.1 mg/kg body weight/dose to about 30 mg/kg body weight/dose, preferably, from about 0.3 mg/kg to about 10 mg/kg. Specific doses such as 10 mg/kg at q2d x 5 regimen (multiple dose) or 30 mg/kg on a single dose regimen can be administered.

In all aspects of the invention where polymeric conjugates are administered, the dosage amount mentioned is based on the amount of 7-ethyl-10-hydroxycamptothecin rather than the amount of polymeric conjugate administered. It is contemplated that the treatment will be given for one or more cycle until the desired clinical result is obtained. The exact amount frequency and period of administration of the compound of the present invention will vary, of course, depending upon the sex, age and medical condition of the patient as well as the severity of the disease as determined by the attending clinician.

Further aspects of the present invention include combining the compounds described herein with other anticancer therapies for synergistic or additive benefit.

### E. TREATMENT OF NEUROBLASTOMA

The present invention provides compounds of formula (I) for use in methods of treatment of neuroblastoma. In one referred aspect, the present invention provides compounds of formula (I) for use in methods of treating patients with neuroblastoma. For purposes of the present invention, "treatment" or "cure" shall be understood to mean inhibition, reduction, amelioration and prevention of tumor growth, tumor burden and metastasis, remission of tumor, or prevention of recurrences of tumor and/or neoplastic growths in patients after completion of treatment.

Treatment is deemed to occur when a patient achieves positive clinical results. For example, successful treatment of neuroblastoma shall be deemed to occur when at least 20% or preferably 30%, more preferably 40 % or higher (i.e., 50%) decrease in tumor growth including other clinical markers contemplated by the artisan in the field is realized when compared to that observed in the absence of the treatment described herein. Other methods for determining changes in a neuroblastoma clinical status resulting from the treatment described herein include: (1) blood and urine tests for levels of catecholamine metabolites such as homovanillic acid (HVA), vanillylmandelic acid (VMA), dopamine and noreinephrine; (2) imaging tests such as X-rays, computed tomography (CT, CAT scan), magnetic resonance imaging (MRI scan), ultrasound, positron emission tomography (PET scan), MIBG scans; (3) biopsies such as tumor biopsy, bone marrow biopsy; (4) immunohistochemistry study using antibody, radioisotope, dye; and complete blood count (CBC).

In a further/alternative aspect, the present invention provides methods of treating neuroblastoma associated with higher levels of an oncogene called MYCN (N-myc gene amplication) and/or lower levels of tumor suppressor genes called TrkA (nerve growth factor receptor) compared to that observed in a mammal without the disease. The present invention also involves in the treatment of neuroblastoma associated with higher levels of ganglioside GD2.

In a further aspect of the present invention, the treatment described herein can be followed by retinoic acid therapy. 13-cis-retinioc acid can be given after completion of the treatment with the compound of Formula (I). The retinoic acid therapy slows the cancer's ability to make more cancer cells, and changes how these cells look and act.

In a still further aspect, the therapy with the compound of Formula (I) can be administered with radiation therapy concurrently or sequentially. In one embodiment, radioactive iodine such as MIBG (meta-iodbenzylguanidine, radioionated with I-131 or I-123) can be provided internally and/or externally. Radiation therapies are also contemplated.

The present invention can be also performed with bone marrow stem cell transplantation, and peripheral blood stem cell transplantation, and with other therapies, i.e., monoclonal antibody therapy.

In a still further aspect of the invention, the methods include treatment of neuroblastoma related to topoismerase I-associated neuroblastoma. Other aspects of the invention include treatment of neuroblastomas which are resistant or refractory to other anticancer agents such as CPT-11, epidermal growth factor receptor antagonists (for example, Erbitux^{®} cetuximab or C225) therapies and combinations thereof.

### EXAMPLES

The following examples serve to provide further appreciation of the invention but are not meant in any way to restrict the effective scope of the invention. The bold-faced numbers, e.g., compound numbers, recited in the Examples correspond to those shown in the figures.
**General Procedures.** All reactions were run under an atmosphere of dry nitrogen or argon. Commercial reagents were used without further purification. All PEG compounds were dried under vacuum or by azeotropic distillation from toluene prior to use. ¹³C NMR spectra were obtained at 75.46 MHz using a Varian Mercury^{®}300 NMR spectrometer and deuterated chloroform and methanol as the solvents unless otherwise specified. Chemical shifts (δ) are reported in parts per million (ppm) downfield from tetramethylsilane (TMS).
**HPLC Method.** The reaction mixtures and the purity of intermediates and final products were monitored by a Beckman Coulter System Gold^{®} HPLC instrument. It employs a ZOBAX^{®} 300SB C8 reversed phase column (150 x 4.6 mm) or a Phenomenex Jupiter^{®} 300A C18 reversed phase column (150 x 4.6 mm) with a multiwavelength UV detector, using a gradient of 10-90 % of acetonitrile in 0.05 % trifluoroacetic acid (TFA) at a flow rate of 1 mL/min.)

### EXAMPLE 1. ^{40k}4arm-PEG-tBu ester (compound 2):

^{40k}4arm-PEG-OH (12.5 g, 1 eq.) was azeotroped with 220 mL of toluene to remove 35 mL of toluene/water. The solution was cooled to 30°C and 1.0 M potassium t-butoxide in t-butanol (3.75 mL, 3eq x 4 =12 eq.) was added. The mixture was stirred at 30°C for 30 min and then t-butyl bromoacetate (0.975 g, 4 eq. x 4 = 16 eq.) was added. The reaction was kept at 30°C for 1hour and then was cooled to 25 °C. 150 mL of ether was slowly added to precipitate product. The resulting suspension was cooled to 17 °C and stayed at 17°C for half hour. The crude product was filtered and the wet cake was washed with ether twice (2 x 125 mL). The isolated wet cake was dissolved in 50 ml of DCM and the product was precipitated with 350 ml of ether and filtered. The wet cake was washed with ether twice (2 x 125 mL). The product was dried under vacuum at 40°C (yield = 98%, 12.25 g). ¹³C NMR (75.4 MHz, CDCl₃): δ 27.71, 68.48-70.71 (PEG), 80.94, 168.97.

### EXAMPLE 2. ^{40k}4arm-PEG acid (compound 3):

^{40k} 4arm-PEG-tBu ester (compound **2**, 12 g) was dissolved in 120 mL of DCM and then 60 mL of TFA were added. The mixture was stirred at room temperature for 3 hours and then the solvent was removed under vacuum at 35 °C. The resulting oil residue was dissolved in 37.5 mL of DCM. The crude product was precipitated with 375 mL of ether. The wet cake was dissolved in 30 mL of 0.5% NaHCO₃. The product was extracted with DCM twice (2 x 150ml). The combined organic layers were dried over 2.5 g of MgSO₄. The solvent was removed under vacuum at room temperature. The resulting residue was dissolved in 37.5 mL of DCM and the product was precipitated with 300 mL of ether and filtered. The wet cake was washed with ether twice (2 x 125ml). The product was dried under vacuum at 40 °C (yield = 90%, 10.75 g). ¹³C NMR (75.4 MHz, CDCl₃): δ 67.93 - 71.6 (PEG), 170.83.

### EXAMPLE 3. TBDPS-(10)-(7-ethyl-10-hydroxycamptothecin) (compound 5):

To a suspension of 7-ethyl-10-hydroxycamptothecin (compound **4**,2.0 g, 5.10 mmol, 1 eq.) in 100 mL of anhydrous DCM were added Et₃N (4.3 mL, 30.58 mmol, 6 eq.) and TBDPSCl (7.8 mL, 30.58 mmol, 6 eq.). The reaction mixture was heated to reflux overnight and then, was washed with a 0.2 N HCl solution (2 x 50 mL), a saturated NaHCO₃ solution (100 mL) and brine (100 mL). The organic layer was dried over MgSO₄, filtered and evaporated under vacuum. The residue was dissolved in anhydrous DCM and precipitated by addition of hexanes. The precipitation with DCM/hexanes was repeated to get rid of excess TBDPSCl. The solids were filtered and dried under vacuum to give 2.09 g of product. (65% yield). ¹H NMR (300 MHz, CDCl₃): δ 0.90 (3 H, t, J = 7.6 Hz), 1.01 (3 H, t, J = 7.3 Hz), 1.17 (9H, s), 1.83-1.92 (2H, m), 2.64 (2H, q, 6.9 Hz), 3.89 (1 H, s, OH), 5.11 (2H, s), 5.27 (1H, d, J =16.1 Hz), 5.72 (1H, d, J =16.4 Hz), 7.07 (2 H, d, J = 2.63 Hz), 7.36-7.49 (7 H, m), 7.58 (1 H, s), 7.75-7.79 (4H, m), 8.05 (1 H, d, J = 9.4 Hz). ¹³C NMR (75.4 MHz, CDCl₃): 5 7.82,13.28, 19.52, 22.86, 26.48, 31.52, 49.23, 66.25, 72.69, 97.25, 110.09, 117.57, 125.67, 126.57, 127.65, 127.81, 130.02, 131.69, 131.97, 135.26, 143.51, 145.05, 147.12, 149.55, 149.92, 154.73, 157.43, 173.72.

### EXAMPLE 4. TBDPS-(10)-(7-ethyl-10-hydroxycamptothecin)-(20)-Gly-Boc (compound 6):

To a 0 °C solution of TBDPS-(10)-(7-ethyl-10-hydroxycamptothecin) (compound **5**, 3.78 g, 5.99 mmol), 1 eq.) and Boc-Gly-OH (1.57 g, 8,99 mmol, 1.5 eq.) in 100 mL of anhydrous DCM was added EDC (1.72 g, 8.99 mmol, 1.5 eq.) and DMAP (329 mg, 2.69 mmol, 0.45 eq.). The reaction mixture was stirred at 0 °C until HPLC showed complete disappearance of the starting material (approx. 1 hour and 45 minutes). The organic layer was washed with a 0.5% NaHCO₃ solution (2 x 50 mL), water (1 x 50 mL), a 0.1 N HCl solution (2 x 50 mL) and brine (1 x 50 mL); and dried over MgSO₄. After filtration and evaporation under vacuum, 4.94 g of crude product were obtained (quantitative yield). The crude solid was used in the next reaction without further purification. ¹H NMR (300 MHz, CDCl₃): δ 0.89 (3 H, t, J = 7.6 Hz), 0.96 (3 H, t, J = 7.5 Hz), 1.18 (9H, s), 1.40 (9H, s), 2.07-2.29 (3H, m), 2.64 (2H, q, 7.5 Hz), 4.01-4.22 (2H, m), 5.00 (1 H, br s), 5.01 (2H, s), 5.37 (1H, d, J = 17.0 Hz), 5.66 (1H, d, J = 17.0 Hz), 7.08 (1 H, d, J = 2.34 Hz), 7.16 (1H, s), 7.37-7.50 (7 H, m), 7.77 (4H, d, J = 7.6 Hz), 8.05 (1 H, d, J = 9.4 Hz). ¹³C NMR (75.4 MHz, CDCl₃): δ 7.52, 13.30, 19.50, 22.86, 26.45, 28.21, 31.64, 42.28, 49.14, 67.00, 76.65, 79.96, 95.31, 110.13, 118.98, 125.75, 126.45, 127.68, 127.81, 130.03, 131.54, 131.92, 135.25, 143.65, 144.91,145.19, 147.08, 149.27, 154.75, 155.14, 157.10, 166.98, 169.17.

### EXAMPLE 5. TBDPS-(10)-(7-ethyl-10-hydroxycamptothecin)-(20)-Gly·HCl (compound 7):

To a solution of TBDPS-(10)-(7-ethyl-10-hydroxycamptothecin)-(20)-Gly-Boc (compound 6, 1 g, 1.27 mmol) in 5 mL anhydrous dioxane was added 5 mL of a 4 M solution of HCl in dioxane. The reaction mixture was stirred at room temperature until HPLC showed complete disappearance of the starting material (1 hour). The reaction mixture was added to 50 mL of ethyl ether and the resulting solid was filtered. The solid was dissolved in 50 mL DCM and washed with brine (pH was adjusted to 2.5 by addition of a saturated NaHCO₃ solution). The organic layer was dried over MgSO₄, filtered and evaporated under vacuum. The residue was dissolved in 5 mL of DCM and precipitated by addition of 50 mL ethyl ether. Filtration afforded 770 mg (84 % yield) final product. ¹H NMR (300 MHz, CDCl₃): δ 0.84 (3 H, t, J = 7.6 Hz), 1.05 (3 H, t, J = 7.3 Hz), 1.16 (9H, s), 2.15-2.30 (3H, m), 2.59 (2H, q, 7.6 Hz), 4.16 (1H, d, J = 17.9 Hz), 4.26 (1H, d, J = 17.9 Hz), 5.13 (2H, s), 5.46 (1H, d, J = 17.0 Hz), 5.60 (1H, d, J = 17.0 Hz), 7.11 (1 H, d, J = 2.34 Hz), 7.30 (1H, s), 7.40-7.51 (6 H, m), 7.56 (1H, dd, J = 2.34, 9.4 Hz), 7.77 (4H, dd, J = 7.6, 1.6 Hz), 7.98 (1 H, d, J = 9.1 Hz). ¹³C NMR (75.4 MHz, CDCl₃): δ 8.09, 13.72, 20.26, 23.61, 26.94, 31.83, 41.01, 50.71, 67.62, 79.51, 97.03, 111.65, 119.69, 127.13, 128.97, 128.99, 129.11, 131.43, 131.96, 133.00, 133.03,136.51, 145.62, 145.81, 147.24, 148.29, 150.58, 156.27, 158.68, 167.81, 168.34.

### EXAMPLE 6. ^{40k} 4arm-PEG-Gly-(20)-(7-ethyl-10-hydroxycamptothecin)-(10)-TBDPS (compound 8):

To a solution of ^{40k}4arm-PEGCOOH (compound **3,**1.4 g, 0.036 mmol, 1 eq.) in 14 mL of anhydrous DCM was added TBDPS-(10)-(7-ethyl-10-hydroxycamptothccin)-(20)-Gly·HCl (compound 7, 207 mg, 0.29 mmol, 2.0 eq. per active site), DMAP (175 mg, 1.44 mmol, 10 eq.) and PPAC (0.85 mL of a 50% solution in EtOAc, 1.44 mmol, 10 eq.). The reaction mixture was stirred at room temperature overnight and then, evaporated under vacuum. The resulting residue was dissolved in DCM and the product was precipitated with ether and filtered. The residue was recrystallized with DMF/IPA to give the product (1.25 g). ¹³C NMR (75.4 MHz, CDCl₃) : δ 7.45, 13.20, 19.39, 22.73, 26.42, 31.67, 40.21, 49.01, 66.83, 95.16, 110.02, 118.83, 125.58, 126.40, 127.53, 127.73, 129.96, 131.49, 131.76, 131.82, 135.12, 143.51, 144.78, 145.13, 146.95, 149.21, 154.61, 156.92, 166.70, 168.46, 170.30.

**EXAMPLE 7. ^{40k}4arm-PEG-Gly(20)-(7-ethyl-10-hydroxycamptothecin)** (compound **9**):

To compound ^{40k}4arm-PEG-Gly-(20)-(7-ethyl-10-hydroxycamptothecin)-(10)-TBDPS (compound **8**, 1.25 g) was added a solution of TBAF (122 mg, 0.46 mmol, 4 eq.) in a 1:1 mixture of THF and a 0.05 M HCl solution (12.5 mL). The reaction mixture was stirred at room temperature for 4 hours and then, extracted with DCM twice. The combined organic phases were dried over MgSO_{4,} filtered and evaporated under vacuum. The residue was dissolved in 7 mL of DMF and precipitated with 37 mL IPA. The solid was filtered and washed with IPA. The precipitation with DMF/IPA was repeated. Finally the residue was dissolved in 2.5 mL of DCM and precipitated by addition of 25 mL of ether. The solid was filtered and dried at 40 °C in vacuum oven overnight (860 mg). ¹³C NMR (75.4 MHz, CDCl₃): δ 7.48, 13.52, 22.91, 31.67, 40.22, 49.12, 66.95, 94.82, 105.03, 118.68, 122.54, 126.37, 128.20, 131.36, 142.92, 144.20, 144.98, 147.25, 148.29, 156.44, 156.98, 166.82, 168.49, 170.39. This NMR data shows no sign of PEG-COOH which indicates that all of the COOH reacted. The loading, as determined by fluorescence detection was found to be 3.9 which is consistent with full loading of the 7-ethyl-10-hydroxycamptothecin on each of the four branches of the polymer. Repeated runs of this experiments at much larger scale yielded consistent results.

### EXAMPLE 8. Boc-(10)-(7-ethyl-10-hydrogycamptothecin) (compound 10):

To a suspension of 7-ethyl-10-hydroxycamptothecin (compound **4**, 2.45 g, 1 eq.) in 250 mL of anhydrous DCM at room temperature under N₂ were added di-tert-butyl dicarbonate (1.764 g, 1.3 eq.) and anhydrous pyridine (15.2 mL, 30 eq.). The suspension was stirred overnight at room temperature. The hazy solution was filtered through celite (10 g) and the filtrate was washed with 0.5 N HCl three times (3 x 150 mL) and a NaHCO₃ saturated solution (1 x 150ml). The solution was dried over MgSO₄ (1.25 g). The solvent was removed under vacuum at 30°C. The product was dried under vacuum at 40°C (yield = 82%, 2.525g) ¹³C NMR (75.4 MHz, CDCl₃) d 173.53, 57.38, 151.60, 151.28, 150.02, 149.70, 147.00, 146.50, 145.15, 131.83, 127.19, 127.13, 124.98, 118.53, 113.88, 98.06, 84.26, 72.80, 66.18, 49.33, 31.62, 27.73, 23.17, 3.98, 7.90.

### EXAMPLE 9. Boc-(10)-(7-ethyl-10-hydroxycamptothecin)-(20)-Ala-Bsmoc (compound 11):

To a solution of Boc-(10)-(7-ethyl-10-hydroxycamptothecin) (compound 10, 0.85g, 1.71 mmol) and Bsmoc-Ala (0.68g, 2.30 mmol) in anhydrous CH₂Cl₂ (20 mL) were added EDC (0.51 g, 2.67 mmol) and DMAP (0.065g, 0.53 mmol) at 0°C. The mixture was stirred at 0°C for 45 min under N₂, then warmed up to room temperature. When completion of the reaction was confirmed by HPLC, the reaction mixture was washed with 1% NaHCO₃ (2 x 50 ml), H₂O (50 mL) and 0.1 N HCl (2 x 50 mL). The organic phase was dried with anhydrous MgSO₄ and filtrated. Solvent was removed under reduced pressure. The resulting solid was dried under vacuum below 40 ° C overnight to give the product of 1.28 g with the yield of 95%. ¹³C NMR (75.4 MHz, CDCl₃) d : 171.16,166.83, 157.16, 154.78, 151.59, 151.33, 149.82, 147.17, 146.68, 145.35, 145.15, 139.08, 136.88, 133.60, 131.83, 130.45, 130.40, 130.33, 127.40, 127.08, 125.32, 125.14, 121.38, 120.01, 114.17, 95.90, 84.38, 77.19, 76.64, 67.10, 56.66, 53.45, 49.96, 49.34, 31.7, 27.76, 17.94, 14.02, 7.53. ESI-MS, 786.20 [M+ H]⁺.

### EXAMPLE 10. Boc-(10)-(7-ethyl-10-hydroxycamptothecin)-(20)-Ala (compound 12):

A solution of Boc-(10)-(7-ethyl-10-hydroxycamptothecin)-(20)-Ala-Bsmoc (compound **11**, 4.2 g, 5.35 mmol) and 4-piperidinopiperidine (1.17g, 6.96 mmol) in anhydrous CH₂Cl₂ (200 ml) was stirred at room temperature for 5 hours. This mixture was then washed with 0.1 N HCl (2 x 40ml), followed by drying the organic layer over anhydrous MgSO₄. This solution was filtered, and the solvent was removed by vacuum distillation to yield 2.8 g of product with purity of 93%, determined by HPLC. This product was further purified by trituration with ether (3 X 20 ml), and then trituration with ethyl acetate (4 x 20ml) to yield 1.52 g (2.70 mmol) with purity 97%. ¹³C NMR (75.4 MHz, CDCl₃) d 168.39, 166.63, 156.98, 151.20, 151.15, 149.69, 146.67, 146.56, 145.37, 144.53, 131.66, 127.1., 124:99, 119.80, 113.82, 96.15, 84.21, 77.67, 67.16, 49.48, 49.06, 31.56, 27.74, 23.14, 15.98, 13.98, 7.57.

### EXAMPLE 11. ^{40k} 4arm-PEG-Ala-(20)-(7-ethyl-10-hydroxycamptothecin)-(10)-Boc (compound 13):

To anhydrous CH₂Cl₂ (100 mL) Boc-(10)-(7-ethyl-10-hydroxycamptothecin)-(20)-Ala (compound **12**, 1.50 g, 2.5 mmol) and 4armPEG-COOH (compound 3, 10.01g, 1.0 mmol) were added at room temperature. The solution was cooled to 0°C, followed by addition of EDC (0.29g, 1.5 mmol) and DMAP (0.30g, 2.5 mmol). The mixture was stirred at 0 °C for 1 hour under N₂. Then it was kept at room temperature overnight. The solvent was evaporated under reduced pressure. The residue was dissolved in 40 mL of DCM, and the crude product was precipitated with ether (300 mL). The wet solid resulting from filtration was dissolved in a mixture of DMF/IPA (60/240 mL) at 65°C. The solution was allowed to cool down to room temperature within 2 ∼ 3 hours, and the product was precipitated. Then, the solid was filtered and washed with ether (2 x 200 mL). The wet cake was dried under vacuum below 40°C overnight to give product of 8.5g.

### EXAMPLE 12. ^{40k} 4arm-PEG-Ala-(20)-(7-ethyl-10-hydroxycamptothecin) (compound 14):

To a solution (130 mL) of 30% TFA in anhydrous CH₂Cl₂ ^{40k}4arm-PEG-Ala-(20)-(7-ethyl-10-hydroxycamptothecin)-(10)-Boc (compound 13, 7.98 g) was added at room temperature. The mixture was stirred for 3 hours, or until the disappearance of starting material was confirmed by HPLC. The solvents were removed as much as possible under vacuum at 35°C. The residues were dissolved in 50mL of DCM, and the crude product was precipitated with ether (350 mL) and filtered. The wet solid was dissolved in a mixture of DMF/IPA (50/200 mL) at 65°C. The solution was allowed to cool down to room temperature within 2 ∼ 3 hours, and the product was precipitated. Then the solid was filtered and washed with ether (2 x 200 mL). The wet cake was dried under vacuum below 40 ° C overnight to give product of 6.7g. ¹³C NMR (75.4 MHz, CDCl₃) d: 170.75, 169.30, 166.65, 157.00, 156.31, 148.36, 147.19, 145.03, 144.29, 143.00, 131.49, 128.26, 126.42, 122.47, 118.79, 105.10, 94.57, 78.08, 77.81, 77.20, 71.15, 70.88, 70.71, 70.33, 70.28, 70.06, 69.93, 69.57, 66.90, 49.14, 47.14, 31.53, 22.95, 17.78, 13.52, 7.46.

### EXAMPLE 13. Boc-(10)-(7-ethyl-10-hydroxycamptothecin)-(20)-Met-Bsmoc (compound 15):

To a solution of Boc-(10)-7-ethyl-10-hydroxycamptothecin (compound **10**, 2.73g, 5.53 mmol) and Bsmoc-Met (3.19 g, 8.59 mmol) in anhydrous CH₂Cl₂ (50 mL) were added EDC (1.64g, 8.59 mmol) and DMAP (0.21 g, 1.72 mmol) at 0°C. The mixture was stirred at 0°C for 45 minutes under N₂, then warmed up to room temperature. When completion of the reaction was confirmed by HPLC, the reaction mixture was washed with 1 % NaHCO₃ (2 x 100 ml), H₂O (100 mL) and 0.1 N HCl (2 x 100 mL). The organic phase was dried with anhydrous MgSO₄ and filtrated. Solvents were removed under reduced pressure. The resulting solid was dried under vacuum below 40°C overnight to give the product of 4.2 g with the yield of 88 %. ¹³C NMR (75.4 MHz, CDCl₃) d: 170.3, 166.8, 157.1, 155.2, 151.4, 151.2, 149.7, 147.0, 146.6, 145.3, 145.1, 138.9, 136.6, 133.5, 131.7, 130.5, 130.3, 130.2, 127.3, 127.0, 125.3, 125.1, 121.2, 119.8, 114.1, 96.1, 84.3, 76.7, 67.0, 56.7, 53.5, 53.4, 49.3, 31.6, 31.0, 29.7, 27.7, 23.1, 15.4, 13.9, 7.4; ESI-MS, 846.24 [M + H]⁺.

### EXAMPLE 14. Boc-(10)-(7-ethyl-10-hydroxycamptothecin)-(20)-Met-NH₂·HCl (compound 16):

A solution of Boc-(10)-(7-ethyl-10-hydroxycamptothecin)-(20)-Met-Bsmoc (compound **15**, 4.1 g, 4.85 mmol) and 4-piperidinopiperidine (1.06 g, 6.31 mmol) in anhydrous CH₂Cl₂ (200 mL) was stirred at room temperature for 5 hours. This mixture was then washed with 0.1 N HCl (2 x 40ml), followed by drying the organic layer over anhydrous MgSO₄. This solution was filtered, and the solvent was removed by vacuum distillation to yield 2.8 g of product with purity of about 97%, determined by HPLC. This product was further purified by trituration with ether (3 x 20 ml), and then trituration with ethyl acetate (4 x 20ml) to yield 1.54 g with purity of 97%. ¹³C NMR (75.4 MHz, CDCl₃) d: 167.2, 166.5, 156.9, 151.12, 150.9, 149.8, 146.3, 145.9, 145.8, 144.9, 131.3, 127.2, 127.0, 125.1, 119.6, 113.8, 96.7, 84.3, 78.2, 67.0, 60.4, 52.2, 49.4, 31.4, 29.6, 29.1, 27.7, 23.2, 15.1, 13.9, 7.7.

### EXAMPLE 15. ^{40k} 4arm-PEG-Met-(20)-(7-ethyl-10-hydroxycamptothecin)-(10)-Boc (compound 17):

To an anhydrous CH₂Cl₂ (80 mL) solution, Boc-(10)-(7-ethyl-10-hydroxycamptothecin)-(20)-Met (compound **16**, 1.48 g, 2.25 mmol) and 4arm-PEG-COOH (compound 3, 9.0 g, 0.9 mmol) were added at room temperature. The solution was cooled to 0°C, followed by addition of EDC (0.26 g, 1.35 mmol) and DMAP (0.27 g, 2.25 mmol). The mixture was stirred at 0°C for 1 hour under N₂. Then it was kept at room temperature overnight The reaction mixture was diluted with 70 ml of CH₂Cl₂, extracted with 30 ml of 0.1 N HCl/1M NaCl aqueous solution. After the organic layer was dried with MgSO₄, the solvent was evaporated under reduced pressure. The residue was dissolved in 40 mL of CH₂Cl₂, and the crude product was precipitated with ether (300 mL). The wet solid resulting from filtration was dissolved in 270 mL of DMF/IPA at 65°C. The solution was allowed to cool down to room temperature within 2 ∼ 3 hours, and the product was precipitated. Then the solid was filtered and washed with ether (2 X 400 mL). The above crystallization procedure in DMF/IPA was repeated. The wet cake was dried under vacuum below 40 °C overnight to give product of 7.0 g. ¹³C NMR (75.4 MHz, CDCl₃) d:169.8, 169.6, 166.5, 156.9, 151.2, 151.1, 149.9, 147.0, 146.6, 145.0, 131.7, 127,1, 126.8, 124.9, 119.7, 113.8, 95.5, 84.1, 70.1, 69.9, 66.9, 50.7, 49.2, 31.5, 31.2, 29.6, 27.6, 23.1, 15.3, 13.9, 7.5.

### EXAMPLE 16. ^{40k} 4arm-PEG-Met-(20)-(7-ethyl-10-hydroxycamptothecin) (compound 18):

To a solution of 30% TFA in anhydrous CH₂Cl₂ (100 mL), dimethyl sulfide (2.5 mL) and 4arm-PEG-Met-(20)-(7-ethyl-10-hydroxycamptothecin)-(10)-Boc (compound 17, 6.0 g) were added at room temperature. The mixture was stirred for 3 hours, or until disappearance of starting material was confirmed by HPLC. Solvents were removed as much as possible under vacuum at 35°C. The residues were dissolved in 50mL of CH₂Cl₂, and the crude product was precipitated with ether (350ml), and filtered. The wet solid was dissolved in a mixture of DMF/IPA (60/300 mL) at 65°C. The solution was allowed to cool down to room temperature within 2 ∼ 3 hours, and the product was precipitated. Then the solid was filtered and washed with ether (2 x 200 mL). The wet cake was dried under vacuum below 40°C overnight to give product of 5.1 g. ¹³C NMR (75.4 MHz, CDCl₃) d :169.7, 166.6, 157.0, 156.3, 148.4, 147.3, 145.0, 144.4, 142.9, 131.5, 128.3, 126.4, 122.5, 118.7, 105.2, 94.7, 78.1, 67.0, 50.7, 49.2, 31.6, 31.3, 29.7, 23.0, 15.3, 13.5, 7.5; Ratio of 7-ethyl-10-hydroxycamptothecin to PEG : 2.1% (wt).

### EXAMPLE 17. Boc-(10)-(7-ethyl-10-hydroxycamptothecin)-(20)-Sar-Boc (compound 19):

Boc-Sar-OH (432 mg, 2.287 mmol) was added to a solution of Boc-(10)-(7-ethyl-10-hydroxycamptothecin) (compound 10, 750 mg, 1.52 mmol) in 75 mL of DCM and cooled to 0 °C. DMAP (432 mg, 2.287 mmol) and EDC (837 mg, 0.686 mmol) were added and the reaction mixture was stirred from 0 °C - room temperature for 1.5 hours. Reaction mixture was then washed with 0.5% NaHCO₃ (75 mL x 2), with water (75 ml x 2) and finally washed with 0.1 N HCl (75 mL x 1). The methylene chloride layer was dried over MgSO₄ and the solvent was evaporated under vacuum and dried. Yield = 0.900 mg.(89%). The structure was confirmed by NMR.

### EXAMPLE 18. 7-ethyl-10-hydroxycamptothecin-(20)-Sar·TFA (compound 20):

Boc-(10)-(7-ethyl-10-hydroxycamptothecin)-(20)-Sar-Boc (compound **19**, 900 mg, 1.357 mmol) was added to a solution of 4 mL TFA and 16 mL DCM, and stirred at room temperature for 1 hour. The reaction mixture was evaporated with toluene at 30°C. The residue was dissolved in 10 mL CHCl₃ and precipitated with ethyl ether. The product was filtered and dried. Yield 700 mg (1.055 mmol, 78%). ¹³C NMR (67.8 MHz, CDCl₃) δ 168.26, 167.07, 158.84, 158.71, 148.82, 147.94, 147.22, 146.34, 144.04, 131.18, 130.08, 128.97, 124.46, 119.78, 106.02, 97.23, 79.84, 79.34, 66.87, 50.84, 49.86, 31.81, 23.94, 15.47, 13.84, 8.08.

### EXAMPLE 19. TBDMS-(10)-(7-ethyl-10-hydroxycamptothecin)-(20)-Sar·HCl (compound 21):

A solution of the 7-ethyl-10-hydroxycamptothecin-(20)-Sar·TFA (compound **20**, 2.17 g, 3.75 mmol, 1 eq.) in anhydrous DMF (30 mL) was diluted with 200 mL of anhydrous DCM. Et₃N (2.4 mL, 17.40 mmol, 4.5 eq.) was added followed by TBDMSC1 (2.04 g, 13.53 mmol, 3.5 eq.). The reaction mixture was stirred at room temperature until HPLC showed disappearance of the starting material (approximately 1 hour). The organic layer was washed with 0.5% NaHCO₃ twice, water once, and a 0.1 N HCl solution saturated with brine twice; and then dried over MgSO₄. After filtration and evaporation of the solvent under vacuum, the resulting oil was dissolved in DCM. Addition of ether gave a solid that was filtered using a fine or medium buchner funnel (2.00 g, 87% yield). HPLC of the solid showed 96% purity. ¹H NMR and ¹³C NMR confirmed the structure. ¹H NMR (300 MHz, CD₃OD): δ 0.23 (6H, s), 0.96 (9H, s), 0.98 (3 H, t, J = 7.3 Hz), 1.30 (3 H, t, J = 7.6 Hz), 2.13-2.18 (2H, m), 2.67 (3H, s), 3.11 (2 H, q, J = 7.6 Hz), 4.10 (1H, d, J = 17.6 Hz), 4.22 (1H, d, J = 17.6 Hz), 5.23 (2 H, s), 5.40 (1 H, d, J = 16.7 Hz), 5.55 (1H, d, J = 16.7 Hz), 7.32 (1H, s), 7.38-7.43 (2H, m), 8.00 (1H, d, J = 9.1 Hz). ¹³C NMR (75.4 MHz, CD₃OD): δ -4.14, 8.01, 14.10, 19.30, 23.98, 26.16, 31.78, 33.52, 49.46, 50.95, 67.66, 79.80, 97.41, 111.96, 119.99, 127.75, 129.28, 129.67, 131.57, 145.24, 146.86, 147.16, 148.02, 150.34, 156.69, 158.72, 167.02, 168.27.

### EXAMPLE 20. ^{40K} 4arm-PEG-Sar-(20)-(7-ethyl-10-hydroxycamptothecin)-(10)-TBDMS (compound 22):

To a solution of ^{40K}4arm-PEG-COOH (compound 3, 10 g, 0.25 mmol, 1 eq.) in 150 mL of anhydrous DCM was added a solution of TBDMS-(10)-(7-ethyl-10-hydroxycamptothecin)-Sar·HCl (compound **21**, 1.53 g, 2.5 mmol, 2.5 eq.) in 20 mL of anhydrous DMF and the mixture was cooled to 0 °C. To this solution were added EDC (767 mg, 4 mmol, 4 eq.) and DMAP (367 mg, 3 mmol, 3 eq.) and the reaction mixture was allowed to warm to room temperature slowly and stirred at room temperature overnight. Then, the reaction mixture was evaporated under vacuum and the residue was dissolved in a minimum amount of DCM. After addition of ether, solid was formed and filtered under vacuum. The residue was dissolved in 30 mL of anhydrous CH₃CN and precipitated by addition of 600 mL IPA. The solid was filtered and washed with IPA and ether to give the product (9.5 g). The structure was confirmed by NMR.

### EXAMPLE 21. ^{40K}4arm-PEG-Sar-(20)-(7-ethyl-10-hydroxycamptothecin) (compound 23):

**Method A**. ^{40K}4arm-PEG-Sar-(20)-(7-ethyl-10-hydroxycamptothecin)-(10) TBDMS (compound **22**) was dissolved in a 50% mixture of TFA in H₂O (200 mL). The reaction mixture was stirred at room temperature for 10 hours and then, diluted with 100 mL of H₂O and extracted with DCM (2 x 300 mL). The combined organic phases were washed with H₂O (2 x 100 mL), dried over MgSO₄, filtered and evaporated under vacuum. The residue was dissolved in 100 mL of anhydrous DMF gently heated with a heat gun and precipitated by slow addition of 400 mL DMF. The solid was filtered and washed with 20% DMF in IPA and ether. The solid was dissolved in DCM and precipitated with ether (6.8 g). The structure was conformed by NMR.
**Method B**. ^{40K}4arm-PEG-Sar-(20)-(7-ethyl-10-hydroxycamptothecin)-(10)-TBDMS (1 g) was dissolved in 10 mL of a IN HCl solution. The reaction mixture was stirred at room temperature for 1 hour (checked by HPLC) and then extracted with DCM (2 x 40 mL). The organic layers were dried over MgSO₄, filtered and evaporated under vacuum. The resulting bright yellow residue was dissolved in 10 mL of DMF (slightly heated with a heat gun) and then 40 mL of IPA were added. The resulting solid was filtered and dried overnight at 40 °C in a vacuum oven. The structure was confirmed by NMR.

### BIOLOGICAL DATA

### EXAMPLE 22. TOXICITY DATA

A maximum tolerated dose ("MTD") of four-arm PEG conjugated 7-ethyl-10-hydroxycamptothecin (compound **9**) as prepared by Example 7, *supra,* was studied using nude mice. Mice were monitored for 14 days for mortality and signs of illness and sacrificed when body weight loss was >20% of the pretreatment body weight.

Table 2, below, shows the maximum tolerated dose of each compound for both single dose and multiple dose administration. Each dose for multiple dose administration was given mice every other day for 10 days and the mice were observed for another 4 days, thus for total 14 days.

**TABLE 2. MTD Data in Nude Mice**

| **Compound** | **Dose Level (mg/kg)** | **Survival/Total** | **Comments** |
|---|---|---|---|
| Compound 9 Single dose | 25 | 5/5 | |
| | 30 | 5/5 | |
| | 35 | 4/5 | Mouse euthanized due to >20% body weight loss |
| Compound 9 | 10 | 5/5 | |
| Multiple dose* | 15 | 3/5 | Mice euthanized due to >20% body weight loss |
| | 20 | 0/5 | Mice euthanized due to >20% body weight loss |

The MTD found for 4arm-PEG-Gly-(7-ethyl-10-hydroxycamptothecin) (compound **9**) was 30 mg/kg when given as single dose, and 10 mg/kg when given as multiple dose (q2d x 5).

### EXAMPLE 23. Properties of PEG Conjugates

Table 3, below, shows solubility of four different PEG-(7-ethyl-10-hydroxycamptothecin) conjugates in aqueous saline solution. All four PEG-(7-ethyl-10-hydroxycamptothecin) conjugates showed good solubility of up to 4 mg/mL equivalent of 7-ethyl-10-hydroxycamptothecin. In human plasma, 7-ethyl-10-hydroxycamptothecin was steadily released from the PEG conjugates with a doubling time of 22 to 52 minutes and the release appeared to be pH and concentration dependent as described in the following EXAMPLE 24.

**TABLE 3. Properties of PEG7-ethyl-10-hydroxycamptothecin Conjugates**

| **Compound** | **Solubility in Saline (mg/mL)^{a}** | **t _{1/2}(min) in Human Plasma^{b}** | **Doubling Time in Plasma (min)^{c}** | | |
|---|---|---|---|---|---|
| | | | **Human** | **Mouse** | **Rat** |
| Compound **9** (Gly) | 180 | 12.3 | 31.4 | 49.5 | 570 |
| Compound **12** (Ala) | 121 | 12.5 | 51.9 | 45.8 | 753 |
| Compound **23** (Sar) | ND | 19.0 | 28.8 | 43.4 | 481 |
| Compound **18** (Met) | 142 | 26.8 | 22.2 | 41.9 | 1920 |

| | | | | | |
|---|---|---|---|---|---|
| ^{a} 7-ethyl-10-hydroxycamptothecin is not soluble in saline. ^{b} PEG conjugate half life. ^{c} 7-ethyl-10-hydroxycamptothecin formation rate from conjugates. | | | | | |

PEG-7-ethyl-10-hydroxycamptothecin conjugates show good stability in saline and other aqueous medium for up to 24 hours at room temperature.

### EXAMPLE 24. Effects Of Concentration and pH on Stability

Based on our previous work, acylation at the 20-OH position protects the lactone ring in the active closed form. The aqueous stability and hydrolysis properties in rat and human plasma were monitored using UV based HPLC methods. 4armPEG-Gly-(7-ethyl-10-hydroxycamptothecin) conjugates were incubated with each sample for 5 minutes at room temperature.

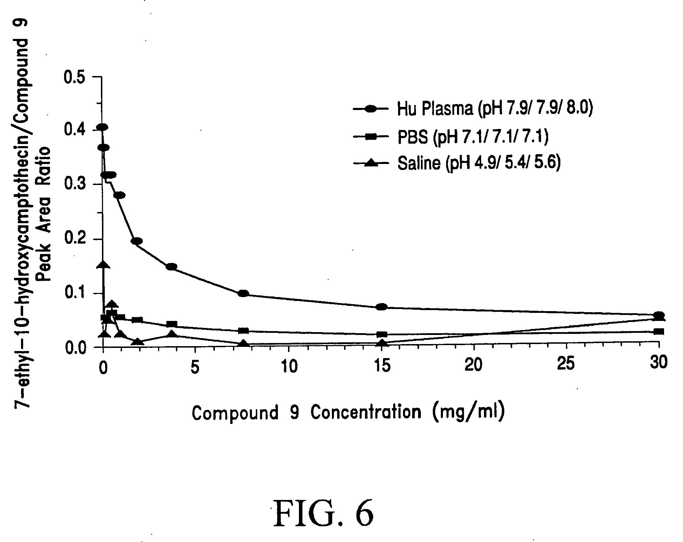

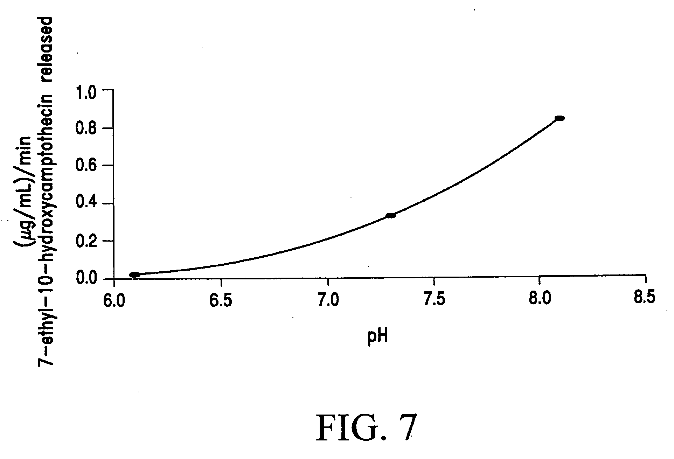

Stability of PEG-7-ethyl-10-hydroxycamptothecin conjugates in buffer was pH dependent. Figure 6 shows 4armPEG-Gly-(7-ethyl-10-hydroxycamptothecin) stability in various samples. Figure 7 shows that rate of 7-ethyl-10-hydroxycamptothecin release from PEG-Gly-(7-ethyl-10-hydroxycamptothecin) increases with increased pH.

### EXAMPLE 25. Pharmacokinetics

Tumor free Balb/C mice were injected with a single injection of 20 mg/kg 4armPEG-Gly-(7-ethyl-10-hydroxycamptothecin) conjugates. At various time points mice were sacrificed and plasma was analyzed for intact conjugates and released 7-ethyl-1 0-hydroxycamptothecin by HPLC. Pharmacokinetic analysis was done using non-compartmental analysis (WinNonlin). Details are set forth in Table 4, below.

**Table 4. Pharmacokinetic Data**

| **Parameter** | **Compound 9** | **7-ethyl-10-hydroxycamptothecin Released from Compound 9** |
|---|---|---|
| **AUC (h*µg/mL)** | 124,000 | 98.3 |
| **Terminal t ½ (Hr)** | 19.3 | 14.2 |
| **Cₘₐₓ (µg/mL)** | 20,500 | 13.2 |
| **CL(mL/hr/kg)** | 5.3 | 202 |
| **Vss (mL/kg)** | 131 | 3094 |

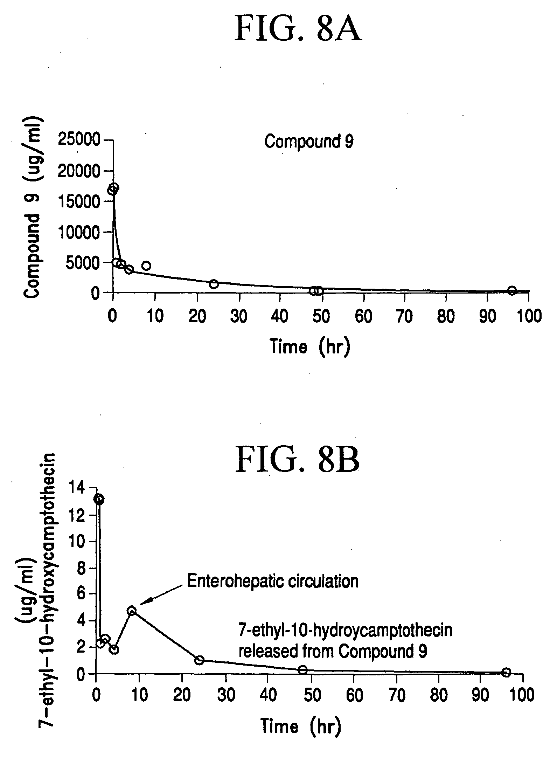

As shown in Figures 8A and 8B, PEGylation of 7-ethyl-10-hydroxycamptothecin allows long circulation half life and high exposure to native drug 7-ethyl-10-hydroxycamptothecin. Enterohepatic circulation of 4armPEG-Gly-(7-ethyl-10-hydroxycamptothecin) conjugates was observed. The pharmacokineric profile of PEG-Gly-(7-ethyl-10-hydroxycamptothecin) in mice was biphasic showing a rapid plasma distribution phase during the initial 2 hours followed by a 18-22 hours terminal elimination half-life for the conjugate and a concomitant 18-26 hours terminal elimination half-life for 7-ethyl-10-hydroxycamptothecin.

Additionally, pharmacokinetic profiles of 4arm PEG-Gly-(7-ethyl-10-hydroxycamptothecin) were investigated in rats. In rats, does levels of 3, 10 and 30 mg/kg (7-ethyl-10-hydroxycamptothecin equivalent) were used. The pharmacokinetic profiles in rats were consistent with those of mice.

In rats, 4arm PEG-Gly-(7-ethyl-10-hydroxycamptothecin) showed a biphasic clearance from the circulation with an elimination half life of 12-18 hours in rats. 7-ethyl-10-hydroxycamptothecin released from 4armPEG-Gly-7-ethyl-10-hydroxycamptothecin conjugates had an apparent elimination half life of 21-22 hours. The maximum plasma concentration (Cₘₐₓ) and area under the curve (AUC) increased in a dose dependent manner in rats. The apparent half life of released 7-ethyl-10-hydroxycamptothecin from 4armPEG-Gly conjugates in mice or rats is significantly longer than the reported apparent half life of released 7-ethyl-10-hydroxycamptothecin from CPT-11 and the exposure of released 7-ethyl-10-hydroxycamptothecin from 4arm PEG-Gly-(7-ethyl-10-hydroxycamptothecin) is significantly higher than the reported exposure of released 7-ethyl-10-hydroxycamptothecin from CPT-11. The clearance of the parent compound was 0.35 mL/hr/kg in rats. The estimated volume of distribution at steady state (Vss) of the parent compound was 5.49 mL/kg. The clearance of the released 7-ethyl-10-hydroxycamptothecin was 131 mL/hr/kg in rats. The estimated Vss of released 7-ethyl-10-hydroxycamptothecin was 2384 mL/kg in rats. Enterohepatic circulation of released 7-ethyl-10-hydroxycamptothecin was observed both in mice and rats.

### EXAMPLE 26. In Vivo DATA-Antitumor Efficacies In Pseudometastatic Human Neuroblastoma (HTLA-230) Xenografted Mice Model

The antitumor efficacy of compound **9** of Example 7 was measured by survival rate in HTLA-230 human neuroblastoma xenografted mice. Xenografts were established in mice by injecting human neuroblastoma cells (HTLA-230) intravenously at zero time point

Four-week-old female athymic nude (nu/nu) mice were purchased from Harlan Laboratories (Harlan Italy-S.Pietro al Natisone, UD). Eight mice/group were injected with the human NB cell line, HTLA-230 (3.5×10⁶ cells in 200 µl of HEPES-buffered saline) in the tail vein (*i.v.*) on day 0. The mice were then randomly assigned to each test group (8 mice per group). Either 24 or 72 hours after the neuroblastoma cell injection, mice received 10 mg/kg body weight (based on SN38) of compound **9** intravenously in the group treated with compound **9** at q2d x 5 (thus, either at day 1, 3, 5 and 9; or at day 3, 5, 7, 9 and 11). In the mice treated with CPT-11, 10 mg/kg body weight of CPT-11 was injected at q2d x 5. Control group received HEPES-buffered saline. The mice were monitored routinely for weight loss. Survival times were used as the criteria for determining treatment efficacy.

In all aspects, the amount of compound **9** administered is based on the amount of 7-ethyl-10-hydroxycamptothecin, not the amount of polymeric conjugate administered.

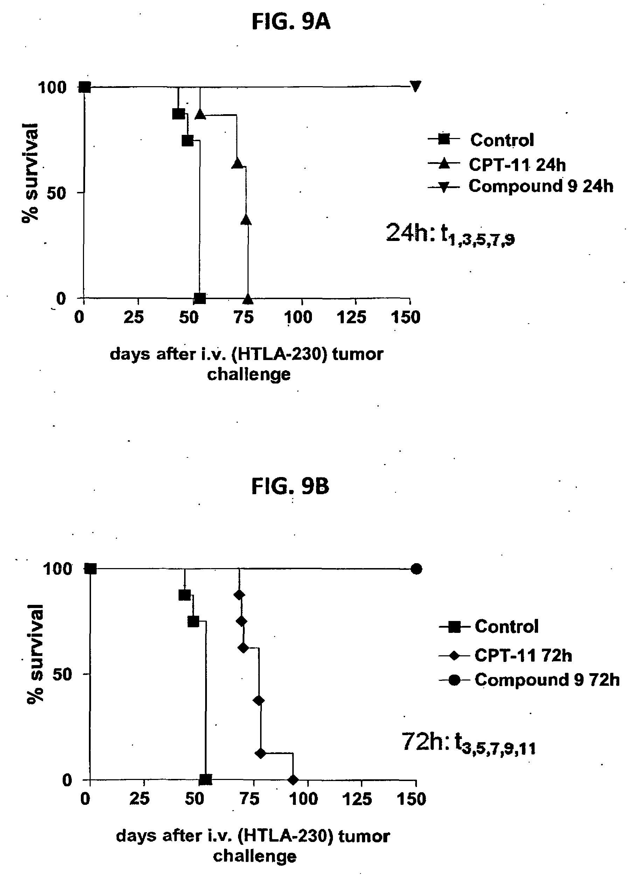

The mice treated with compound **9,** both 24 and 72 hours after the neuroblastoma cell injection, displayed significantly increased life span compared to the control mice or those treated with CPT-11. The results of the survival rate of the treatment 24 or 72 hours after the neuroblastoma cell injection are set forth in FIG. 9A and FIG.9B.

The results show that the mice treated with compound **9** had 100 % survival rate 150 days after the cancer injection. None of the control mice or mice treated with CPT-11 survived. The control and CPT-11-treated animals died within 50 and 85 days, respectively from the cancer injection because of the metastatic cancer.

The HTLA-230 cells are a human neuroblastoma cell line isolated from a patient with advanced disease. Details of the cancer cell can be found in Bogenmann, Int. J. Cancer, 1996, 67:379-85, the contents of which are incorporated herein by reference.

The mice xenografted with HTLA-230 are biologically and clinically relevant to the neuroblastoma in the pseudometastatic stage of the disease. This model is also relevant to the stage of neuroblastoma which includes cells that exist or survive after treatment with some modality such as radiotherapy and chemotherapy.

When HTLA-230 neuroblastoma cells are injected intravenously in the mice, this xenograft model mimics the metastatic spread observed in advanced stage NB patients. *See* Bogenmann. E., 1996, Int. J. Cancer, 67: 381-386, 1996; and Pastorino F. et al., Cancer Res. 2003; 63: 86-92.

A number of studies conducted on large cohorts of patients have shown that the presence of circulating neuroblastoma cells in the blood and micrometastases in the bone marrow at the time of primary surgery is a strong predictor of relapse. Moss, T. et al., 1991, New Engl. J. Med., 324: 219-226. Since bone marrow micrometastases are a direct measurement of the ability of tumor cells to spread systemically, this model, which closely mimics the clinical situation, allows a more realistic evaluation of antitumor therapies and thus provides evidence in favor of the use of compound **9** in the treatment of neuroblastoma. The schedule of treatment was deliberately chosen to allow evaluation of the effects of treatments during the metastatic cascade, i. e., during the stages in which tumor cells are in intravascular circulation and endothelium-attachment take place or when extravasation, stromal invasion and colonization take place. *See* Al-Mehbi, A. B. et al., Nature Med., 2000, 6: 100-102; and Chambers, A. F. et al., Nature Rev. Cancer, 2002, 2: 563-572.

The results indicate that the compounds described herein have advantages over therapy based on CPT-11.

### EXAMPLE 27. In Vivo DATA-Antitumor Efficacies In orthotopic Human Neuroblastoma (GI-LI-N) Xenografted Mice Model

The antitumor efficacy of compound **9** was measured in orthotopic human neuroblastoma xenografted mice. Xenograft tumors were established in mice by injecting human neuroblastoma cells (GI-LI-N) in the adrenal gland.

Five-week-old female athymic nude (nu/nu) mice were randomly assigned to each test group (8 mice per group). Eight mice/group were anaesthetised and injected with the human NB cell line, GI-LI-N (1 × 10⁶ cells in 15 µl of HEPES buffer), after laparatomy, in the capsule of the left adrenal gland. Mice were monitored at least two times weekly for evidence of tumor development, quantification of tumor size, and evidence of tumor-associated morbidity. Tumors were allowed to grow for 20 days and reached the average volume of approximately 200 mm³. Then, 10 mg/kg body weight of compound **9** was injected intravenously in the group treated with compound **9** at day 21, 23, 25, 27 and 29 after the tumor cell injection. In the mice treated with CPT-11, 10 mg/kg body weight of CPT-11 was injected at q2d x 5. Control group received HEPES-buffered saline. The body weight and general physical status of the animals was observed daily and any mouse was terminated when a tumor reached 1000-1200 mm³. Survival time was used as the criteria for determining treatment efficacy.

In these experiments, the amount of compound **9** administered was based on the amount of 7-ethyl-10-hydroxycamptothecin, not the amount of polymeric conjugate administered.

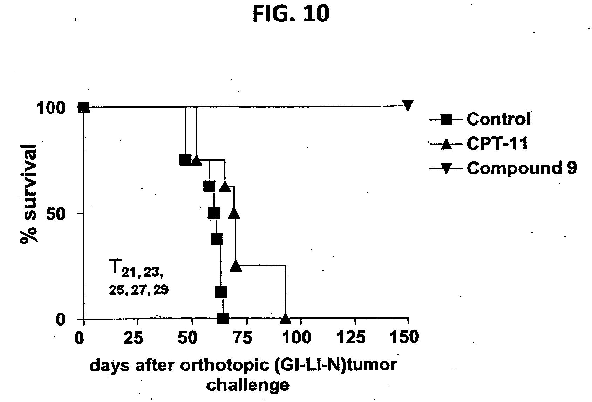

The mice treated with compound **9** displayed significantly increased life span compared to the control mice or those treated with CPT-11. The results of survival rate are set forth in FIG. 10.

The results show that the mice treated with compound **9** had 100 % survival rate 100 days after the tumor cell implantation. None of the control mice or mice treated with CPT-11 survived. Both the control and CPT-11-treated animals died within 80 days because of the cancer. The mice treated with compound **9** showed a dramatic arrest and regression in the growth of primary tumors as compared to the control mice.

The mice xenografted with GI-LI-N are clinically relevant to human neuroblastoma at the stage with a massive tumor (solid tumor) and metastases. The orthotopic implant model includes treatment of a large primary mass that metastasizes. The results indicate that the treatment described herein has utility in treating patients with neuroblastoma in the later or advanced stage. The results also indicate that the compounds described herein are an alternative to therapy based on CPT-11.

The GI-LI-N cell xenografted model is a reproducible, angiogenic, and metastatic orthotopic model ofNB. This model reflects the typical growth pattern of human NB, since orthotopic injection of NB cells resulted in solid adrenal tumors that were highly vascular, locally invasive into surrounding tissues, and metastatic to distant sites. It is also reported that macroscopic metastases occurred after 3-4 weeks of injection in the contralateral adrenal and liver while micrometastases were apparent in the ovary, right kidney, liver, and lung (6-10). See Pastorino F. et al., Cancer Res. 2003, 63: 7400-7409; Brignole C. et al., J Natl Cancer Inst. 2006, 98:1142-57; Marimpietri D. et al., Clin Cancer Res. 2007, 13: 3977-3988; Pastorino F. et al., Current Medicinal Chemistry 2007,14:3070-8; and Pastorino F. et al., Clinical Cancer Research, 2008, 14:7320-7329.

The results described in Examples 26 and 27 indicate that the treatments described herein have utility in treating patients in various stages of neuroblastoma.

### EXAMPLE 28. In Vivo DATA-Efficacies In GI-LI-N Xenografted Mice Model

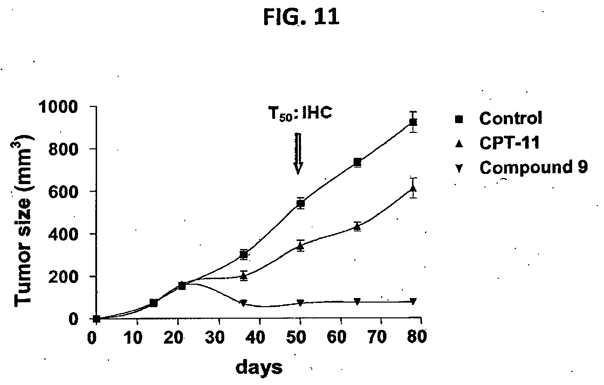

In mice xenografted with GI-LI-N neuroblastoma tumor cells described in Example 27, tumor size was measured at various time points. The results are set forth in FIG. 11.

The orthotopic neuroblastoma xenografted mice treated with compound **9** showed a dramatic arrest and regression in the primary tumor growth compared to control mice or those treated with CPT-11. Compound **9** is significantly more effective than CPT-11 in the treatment of the metastatic cancer.

### EXAMPLE 29. In Vivo DATA- Efficacies In GI-LI-N Xenografted Mice Model

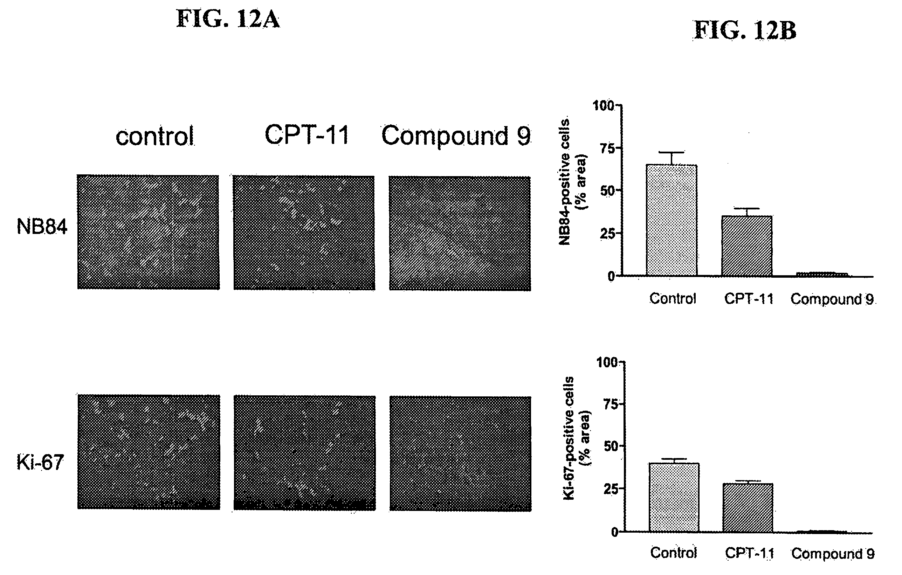

Histological examination of tumors was performed in the mice described in Example 27. Tumors were removed from orthotopic neuroblastoma-bearing mice at day 50. Tumor sections were immunostained for NB84 as a neuroblastoma cell marker, and Ki-67 as a tumor cell proliferation marker. Cell nuclei were stained with DAPI. Immunostained tumor sections and positive cells for each group of the mice are shown in FIG. 12A, and measurements are shown in FIG. 12B.

The histological study confirms that the mice treated with compound 9 were free or nearly free of neuroblastoma marker-positive cells. The data confirms that the tumor cells disappeared in the mice by the treatment described herein.

### EXAMPLE 30. In Vivo DATA-Comparison Study with CPT-11 at MTD In GI-LI-N Xenografted Mice Model

As described in Example 27, human neuroblastoma cells, GI-LI-N, were implanted in the left adrenal gland of immunodeficient nude mice. Tumors were allowed to grow for 20 days and reached the average volume of approximately 200 mm³. 10 mg/kg body weight of compound **9** (based on SN38) was injected intravenously in the group treated with compound **9** at day 21, 23, 25, 27 and 29 after the tumor cell injection. In the mice treated with CPT-11, 10 or 40 mg/kg body weight (MTD) of CPT-11 was injected at day 21, 23, 25, 27 and 29. Control group received HEPES-buffered saline.

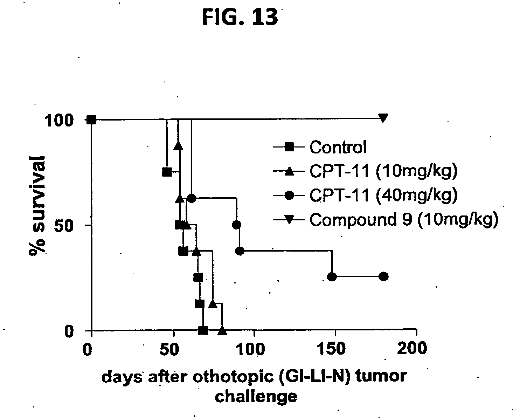

The mice treated with compound **9** displayed significantly increased life span compared to the control mice or those treated with CPT-11. The results of survival rate are set forth in FIG. 13.

The results show that the mice treated with compound 9 had 100 % survival rate 180 days after the tumor cell implantation. None of the control mice or mice treated with CPT-11 at 10 mg/kg/dose survived. The animals died within 80 days because of the cancer. The treatment with CPT-11 at MTD (40 mg/kg) slightly improved survival rate. The therapeutic efficacy of compound **9** was greater than therapy with CPT-11 at MTD.

### EXAMPLE 31. In Vivo DATA- Efficacies in NXS2 Xenografted Mice Model

The antitumor efficacy of compound **9** was measured in mice xenografted with human neuroblastoma cells NXS2. Xenograft tumors were established in immunocompetent A/J mice by injecting human neuroblastoma cells, NXS2, in the adrenal gland.

The immunocompetent mice were injected with another human NB cell, NXS2 of 5 x 10⁴ cells or 5 x 10⁵ cells in the left adrenal gland. Tumors were allowed to grow for 2 days and reached the average volume of approximately 200 mm³. Then, 10 mg/kg body weight of compound **9** was injected intravenously in the group treated with compound **9** at day 3, 5, 7, 9, and 11 after the tumor cell injection. In the mice treated with CPT-11, 10 or 40 mg/kg body weight of CPT-11 was injected at q2d x 5. Control group received HEPES-buffered saline. Survival time was used as the criteria for determining treatment efficacy.

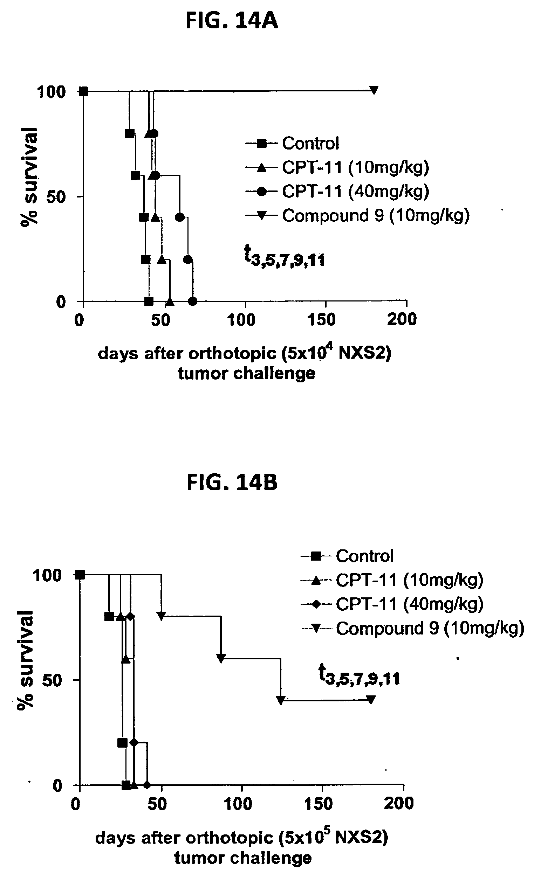

The NXS2 xenografted animal model represents a very aggressive human neuroblastoma as shown in the rapid growth of tumor. The results show that compound **9** was effective in the treatment of the aggressive neuroblastoma. 100 % of the mice treated with compound **9** survived when the mice were challenged with 5 x 10⁴ tumor cells and about 40% of the mice still survived when challenged with higher concentration of 5 x 10⁵ tumor cells. The results of survival rate are set forth in FIG. 14A and FIG. 14B. None of the control mice or mice treated with CPT-11 at 10 mg/kg/dose or MTD survived. The survival rate of the mice treated with CPT-11 was as low as that of the control group. Both CPT-11 therapies with 10mg/kg/dose or MTD were ineffective in the treatment of aggressive neuroblastoma.

### EXAMPLE 32. In Vivo DATA-Antitumor Effects in SH-SY5Y Xenografted Mice Model

Antitumor effects of compound **9** were compared to CAMPTOSAR (CPT-11 in pharmaceutical formulation) in the SH-SY5Y xenografted mice model.

Human neuroblastoma cells, SH-SY5Y, were injected subcutaneously in the right flank of SCID mice at day 0. The SH-SY5Y cells were human neuroblastoma cells transfected with luciferase. The mice were treated with 10 mg/kg/dose of CAMPTOSAR or equivalent dose of compound **9** (based on SN38) one week after the tumor implantation every other day for total 5 doses.

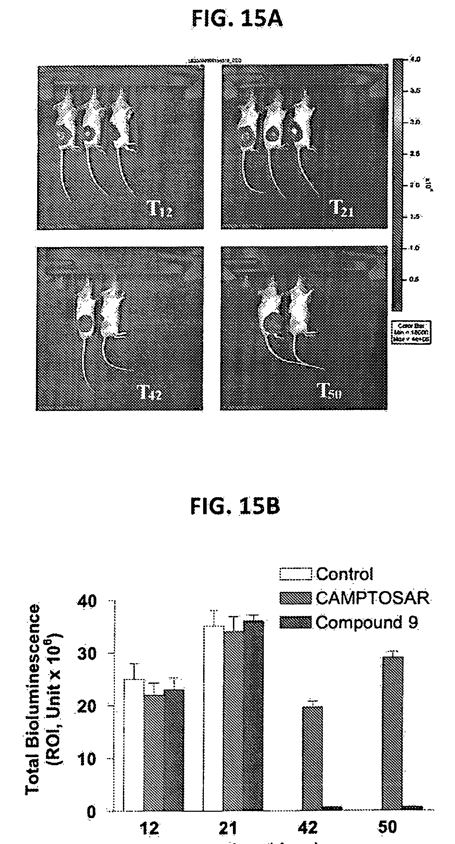

The luciferase-expressing neuroblastoma cells were visualized by bioluminescence imaging (BLI). Lateral images showing the site of tumor implantation were taken at day 0 (T₀), and over time (T₁₂₋₅₀). The BLI images taken at day 12 (T₁₂), day 21 (T₂₁), day 42 (T₄₂) and day 50 (T₅₀) are shown in FIG. 15A. In each image, the first, second and third slots are assigned for a mouse which did not receive any treatment, a mouse treated with CAMPTOSAR and a mouse treated with compound **9.** The BLI intensity increases as the BLI color grades from blue to red. Less luminescence meant less neuroblastoma cells in the mice. Antitumor effects were evaluated by changes in luminescence.

The results show that there were very few tumor cells in the mice treated with compound **9** at day 42 and 50. The luminescent neuroblastoma cells did not decrease in the mice treated with CAMPTOSAR at day 50.

The neuroblastoma cells were quantified based on luminescence. The results are set forth in FIG. 15B. Primary tumor regression was shown in the mice treated with compound **9.** CAMPTOSAR did not treat tumors.

## Claims

1. A compound of Formula (I) for use in a method of treatment of neuroblastoma comprising administering the compound of Formula (I) to a mammal in need thereof, wherein the compound of Formula (I) comprises: 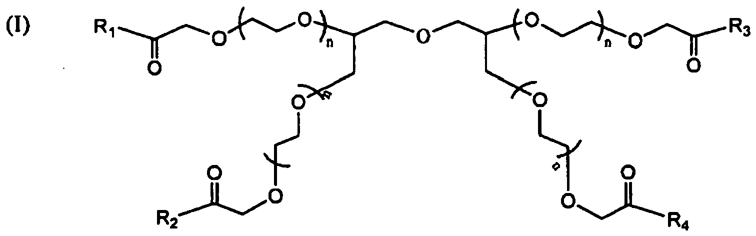 wherein
R₁, R₂, R₃ and R₄ are independently OH or 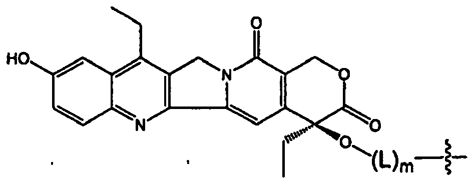 wherein
L is a bifunctional linker;
(m) is a positive integer, wherein each L is the same or different when (m) is equal to or greater than 2; and
(n) is a positive integer;
provided that R₁, R₂, R₃ and R₄ are not all OH;
or a pharmaceutically acceptable salt thereof.

2. The compound for use in a method for the treatment according to claim 1, wherein (m) is from 1 to 10, optionally wherein (m) is 1.

3. The compound for use in a method for the treatment according to claim 1, wherein (n) is from 28 to 341 so that the total molecular weight of the polymeric portion of the compound ranges from 5,000 to 60,000 daltons, optionally wherein (n) is from 114 to 239 so that the total molecular weight of the polymeric portion of the compound ranges from 20,000 to 42,000 daltons, further optionally wherein (n) is 227 so that the total molecular weight of the polymeric portion of the compound is 40,000 daltons.

4. The compound for use in a method for the treatment according to claim 1, wherein R₁, R₂, R₃ and R₄ are all:

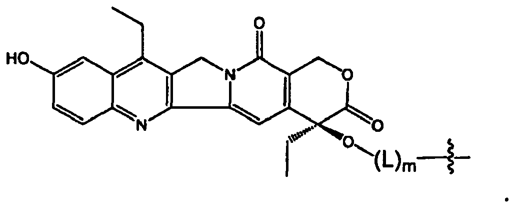

5. The compound for use in a method for the treatment according to claim 1, wherein the compound of Formula (I) is selected from the group consisting of 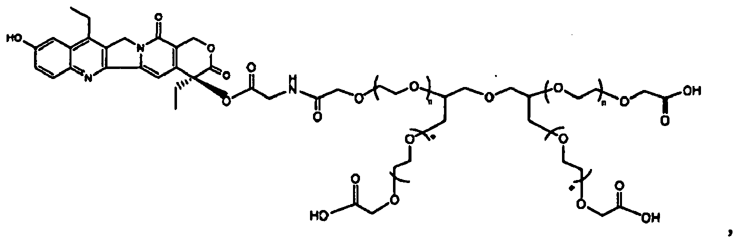 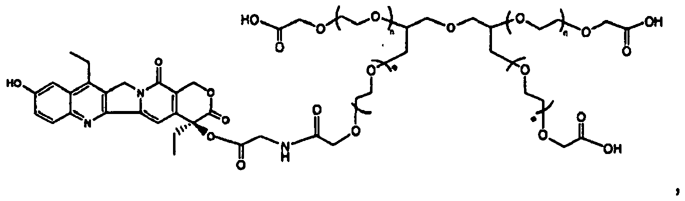 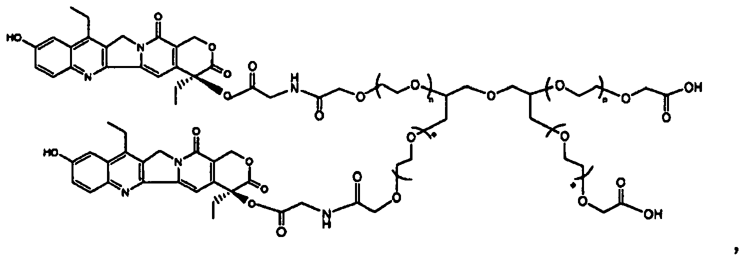 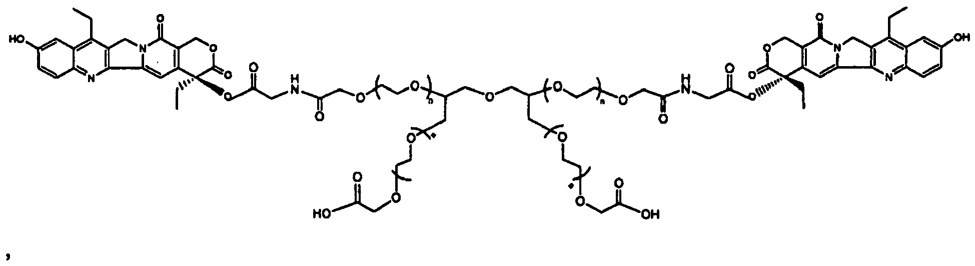 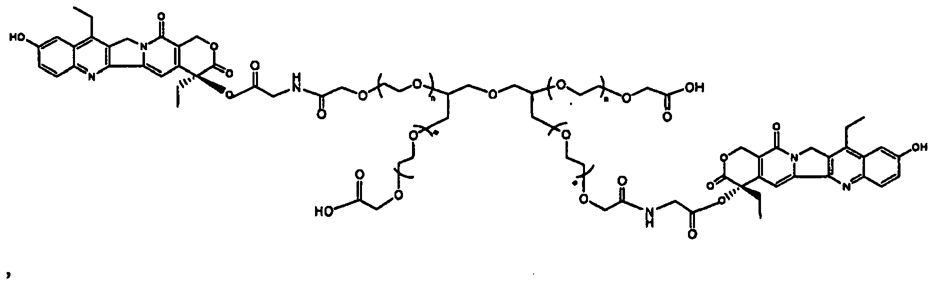 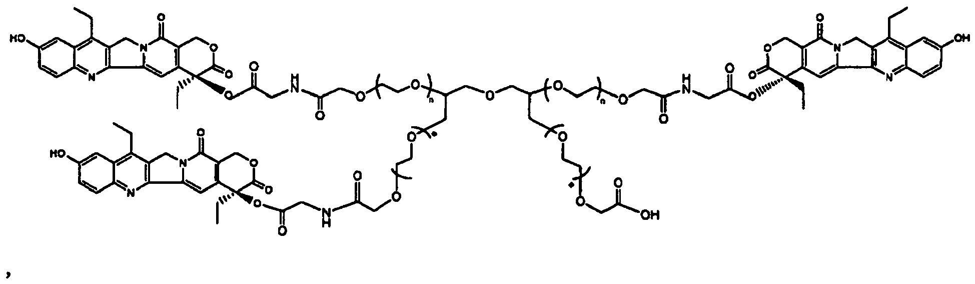 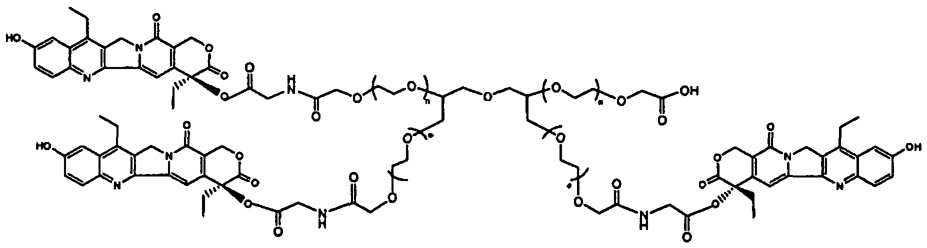 , and

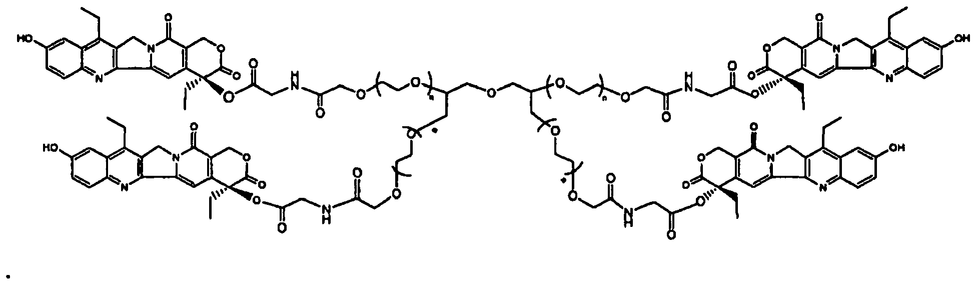

6. The compound for use in a method for the treatment according to claim 1, wherein the compound of Formula (I) is administered in amounts of from 0.5 mg/m² body surface/dose to 50 mg/m² body surface/dose, and wherein the amount is the weight of 7-ethyl-10-hydroxycamptothecin included in the compound of Formula (I), optionally in amounts of from 1 mg/m² body surface/dose to 18 mg/m² body surface/dose, and wherein the amount is the weight of 7-ethyl-10-hydroxycamptothecin included in the compound of Formula (I).

7. The compound for use in a method for the treatment according to claim 1, wherein the compound of Formula (I) is administered according to a protocol of from 1.25 mg/m² body surface/dose to 16.5 mg/m² body surface/dose given weekly for three weeks, followed by 1 week without treatment, and the amount is the weight of 7-ethyl-10-hydroxycamptothecin included in the compound of Formula (I), optionally wherein the amount administered weekly is 5 mg/m² body surface/dose, and the amount is the weight of 7-ethyl-10-hydroxycamptothecin included in the compound of Formula (I).

8. The compound for use in a method for the treatment according to claim 1, wherein the cancer is metastatic, optionally wherein the cancer is a solid tumor.

9. The compound for use in a method for the treatment according to claim 1, wherein the compound of Formula (I) is administered in combination with a second chemotherapeutic agent simultaneously or sequentially, further optionally wherein the compound of Formula (I) is administered, followed by 13-cis-retinioc acid.

10. The compound for use in a method for the treatment according to claim 1, wherein the compound of Formula (I) is administered in combination with radiotherapy simultaneously or sequentially.

11. The compound for use in a method for the treatment according to claim 1, wherein the cancer is resistant or refractory to an anti-cancer therapy that does not include a compound of Formula (I), and optionally wherein the cancer is resistant to an anti-cancer agent that is chosen from camptothecin, CPT-11, an epidermal growth factor receptor antagonist, and combinations thereof, and further optionally wherein the epidermal growth factor receptor antagonist is cetuximab.

12. A compound having the formula: 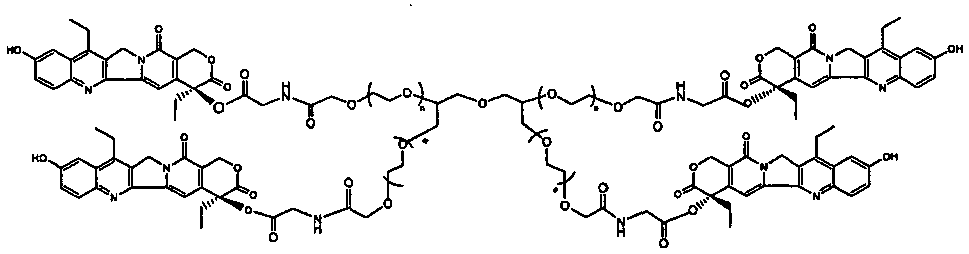 or a pharmaceutically acceptable salt thereof, for use in a method of treatment of neuroblastoma in a mammal in need thereof, comprising administering to said mammal
the compound in amounts of from 1 mg/m² body surface/dose to 18 mg/m² body surface/dose and the amount is the weight of 7-ethyl-10-hydroxycamptothecin included in the compound; and
(n) is 227.

13. The compound for use in a method for the treatment according to claim 12, wherein the compound is administered according to a protocol of from 1.25 mg/m² body surface/dose to 16.5 mg/m² body surface/dose given weekly for three weeks, followed by 1 week without treatment; and the amount is the weight of 7-ethyl-10-hydroxycamptothecin included in the compound, optionally wherein the amount administered weekly is 5 mg/m² body surface/dose, and the amount is the weight of 7-ethyl-10-hydroxycamptothecin included in the compound.

14. The compound for use in a method for the treatment according to claim 1, wherein L is an amino acid or amino acid derivative, wherein the amino acid derivative is selected from the group consisting of 2-aminoadipic acid,
3-aminoadipic acid, beta-alanine, beta-aminopropionic acid, 2-aminobutyric acid,
4-aminobutyric acid, piperidinic acid, 6-aminocaproic acid, 2-aminoheptanoic acid,
2-aminoisobutyric acid, 3-aminoisobutyric acid, 2-aminopimelic acid, 2,4-aminobutyric acid, desmosine, 2,2-diaminopimelic acid, 2,3-diaminopropionic acid, N-ethylglycine,
N-ethylasparagine, 3-hydroxyproline, 4-hydroxyproline, isodesmosine, alloisoleucine,
N-methylglycine, sarcosine, N-methyl-isoleucine, 6-N-methyl-lysine, N-methylvaline, norvaline, norleucine, and ornithine, optionally wherein L is glycine, alanine, methionine or sarcosine.

15. The compound for use in a method for the treatment according to claim 1, wherein L is selected from the group consisting of
-[C(=O)]ᵥ(CR₂₂R₂₃)ₜ- ,
-[C(=O)]ᵥ(CR₂₂R₂₃)ₜ-O- ,
-[C(=O)]ᵥ(CR₂₂R₂₃)ₜ-NR₂₆- ,
-[C(=O)]ᵥO(CR₂₂R₂₃)ₜ- ,
-[C(=O)]ᵥO(CR₂₂R₂₃)ₜO- ,
-[C(=O)]ᵥO(CR₂₂R₂₃)ₜNR₂₆₋ ,
-[C(=O)]ᵥNR₂₁(CR₂₂R₂₃)ₜ- ,
-[C(=O)]ᵥNR₂₁(CR₂₂R₂₃)ₜO- ,
-[C(=O)]ᵥNR₂₁(CR₂₂R₂₃)ₜNR₂₆- ,
-[C(=O)]ᵥ(CR₂₂R₂₃O)ₜ- ,
-[C(=O)]ᵥO(CR₂₂R₂₃O)ₜ- ,
-[C(=O)]ᵥNR₂₁(CR₂₂R₂₃O)ₜ- ,
[C(=O)]ᵥ(CR₂₂R₂₃O)ₜ(CR₂₄R₂₅)_{y}- ,
-[C(=O)ᵥO(CR₂₂R₂₃O)ₜ(CR₂₄R₂₅)_{y}-,
-[C(=O)]ᵥNR₂₁(CR₂₂R₂₃O)ₜ(CR₂₄R₂₅)_{y}-,
-[C(=O)]ᵥ(CR₂₂R₂₃O)ₜ(CR₂₄R₂₅)_{y}O- ,
-[C(=O)]ᵥ(CR₂₂R₂₃)ₜ(CR₂₄R₂₅O)_{y}-,
-[C(=O)]ᵥO(CR₂₂R₂₃O)ₜ(CR₂₄R₂₅)_{y}O- ,
-[C(=O)]ᵥO(CR₂₂R₂₃)ₜ(CR₂₄R₂₅O)_{y}- ,
-[C(=O)]ᵥNR₂₁(CR₂₂R₂₃O)ₜ(CR₂₄R₂₅)_{y}O- ,
-[C(=O)]ᵥNR₂₁(CR₂₂R₂₃)ₜ(CR₂₄R₂₅O)_{y}- ,
-[C(=O)]ᵥ(CR₂₂R₂₃)ₜO-(CR₂₈R₂₉)_{t'}- ,
-[C(=O)]ᵥ(CR₂₂R₂₃)ₜNR₂₆-(CR₂₈R₂₉)_{t'}-,
-[C(=O)]ᵥ(CR₂₂R₂₃)ₜS-(CR₂₈R₂₉)_{t'}-,
-[C(=O)]ᵥO(CR₂₂R₂₃)ₜO-(CR₂₈R₂₉)_{t'}-,
-[C(=O)]ᵥO(CR₂₂R₂₃)ₜNR₂₆-(CR₂₈R₂₉)_{t'}- ,
-[C(=O)]ᵥO(CR₂₂R₂₃)ₜS-(CR₂₈R₂₉)_{t'}-,
-[C(=O)]ᵥNR₂₁(CR₂₂R₂₃)ₜO-(CR₂₈R₂₉)_{t'}-,
-[C(=O)]ᵥNR₂₁(CR₂₂R₂₃)ₜNR₂₆-(CR₂₈R₂₉)_{t'}-,
-[C(=O)]ᵥNR₂₁(CR₂₂R₂₃)ₜS-(CR₂₈R₂₉)_{t'}-,
-[C(=O)ᵥ(CR₂₂R₂₃CR₂₈R₂₉O)ₜNR₂₆-,
-[C(=O)]ᵥ(CR₂₂R₂₃CR₂₈R₂₉O)ₜ-,
-[C(=O)]ᵥO(CR₂₂R₂₃CR₂₈R₂₉O)ₜNR₂₆-,
-[C(=O)]ᵥO(CR₂₂R₂₃CR₂₈R₂₉O)ₜ ,
-[C(=O)]ᵥNR₂₁(CR₂₂R₂₃CR₂₈R₂₉O)ₜNR₂₆-,
-[C(=O)]ᵥNR₂₁(CR₂₂R₂₃CR₂₈R₂₉O)ₜ-,
-[C(=O)]ᵥ(CR₂₂R₂₃CR₂₈R₂₉O)ₜ(CR₂₄R₂₅)_{y}-,
-[C(=O)]ᵥO(CR₂₂R₂₃CR₂₈R₂₉O)ₜ(CR₂₄R₂₅)_{y}- ,
-[C(=O)]ᵥNR₂₁(CR₂₂R₂₃CR₂₈R₂₉O)ₜ(CR₂₄R₂₅)_{y}- ,
-[C(=O)]ᵥ(CR₂₂R₂₃CR₂₈R₂₉O)ₜ(CR₂₄R₂₅)_{y}O- ,
-[C(=O)]ᵥ(CR₂₂R₂₃)ₜ(CR₂₄R₂₅CR₂₈R₂₉O)_{y}- ,
-[C(=O)]ᵥ(CR₂₂R₂₃)ₜ(CR₂₄R₂₅CR₂₈R₂₉O)_{y}NR₂₆-,
-[C(=O)]ᵥO(CR₂₂R₂₃CR₂₈R₂₉O)ₜ(CR₂₄R₂₅)_{y}O-,
-[C(=O)]ᵥO(CR₂₂R₂₃)ₜ(CR₂₄R₂₅CR₂₈R₂₉O)_{y}-,
-[C(=O)]ᵥO(CR₂₂R₂₃)ₜ(CR₂₄CR₂₅CR₂₈R₂₉O)_{y}NR₂₆-,
-[C(=O)]ᵥNR₂₁(CR₂₂R₂₃CR₂₈R₂₉O)ₜ(CR₂₄R₂₅)_{y}O- ,
-[C(=O)]ᵥNR₂₁(CR₂₂R₂₃)ₜ(CR₂₄R₂₆CR₂₈R₂₉O)_{y}- ,
-[C(=O)]ᵥNR₂₁(CR₂₂R₂₃)ₜ(CR₂₄R₂₅CR₂₈R₂₉O)yNR₂₆- , 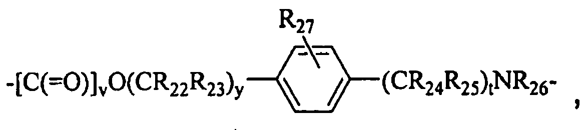 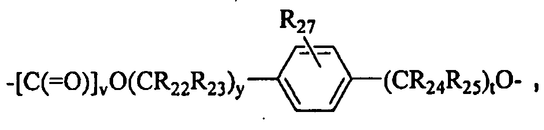 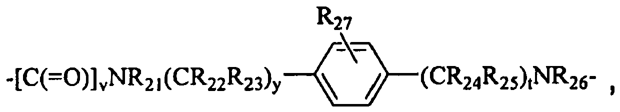 and 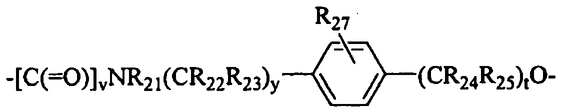 wherein:
R₂₁-R₂₉ are independently selected from the group consisting of hydrogen, amino, substituted amino, azido, carboxy, cyano, halo, hydroxyl, nitro, silyl ether, sulfonyl, mercapto, C₁₋₆ alkylmercapto, arylmercapto, substituted arylmercapto, substituted
C₁₋₆ alkylthio, C₁₋₆ alkyls, C₂₋₆ alkenyl, C₂₋₆ alkynyl, C₃₋₁₉ branched alkyl, C₃₋₈ cycloalkyl,
C₁₋₆ substituted alkyl, C₂₋₆ substituted alkenyl, C₂₋₆ substituted alkynyl, C₃₋₈ substituted cycloalkyl, aryl, substituted aryl, heteroaryl, substituted heteroaryl, C₁₋₆ heteroalkyl, substituted C₁₋₆ heteroalkyl, C₁₋₆ alkoxy, aryloxy, C₁₋₆ heteroalkoxy, heteroaryloxy,
C₂₋₆ alkanoyl, arylcarbonyl, C₂₋₆ alkoxycarbonyl, aryloxycarbonyl, C₂₋₆ alkanoyloxy, arylcarbonyloxy, C₂₋₆ substituted alkanoyl, substituted arylcarbonyl, C₂₋₆ substituted alkanoyloxy, substituted aryloxycarbonyl, C₂₋₆ substituted alkanoyloxy, substituted and arylcarbonyloxy;
(t), (t') and (y) are independently selected from zero or a positive integer; and
(v) is 0 or 1.

## Patentansprüche

1. Verbindung der Formel (I) zur Verwendung in einem Verfahren zur Behandlung von Neuroblastomen, umfassend die Verabreichung der Verbindung der Formel (I) an einen Säuger, der diese benötigt, wobei die Verbindung der Formel (I) umfasst: 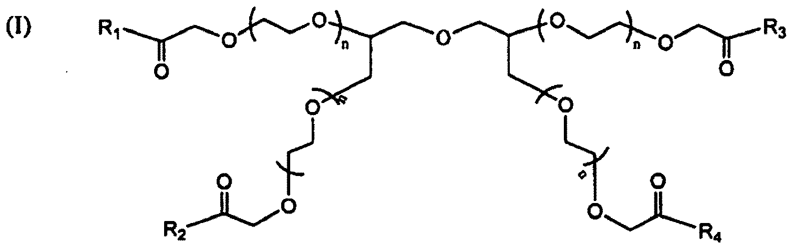 wobei
R₁, R₂, R₃ und R₄ unabhängig OH sind, oder 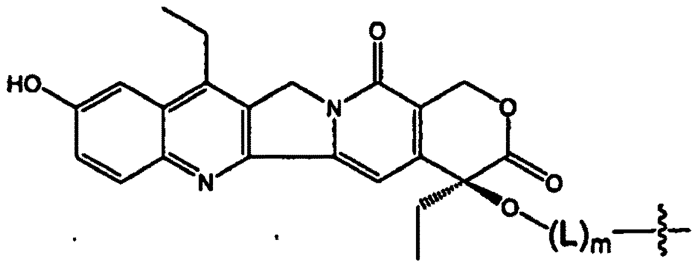 wobei
L ein bifunktioneller Linker ist;
(m) eine positive Ganzzahl ist, wobei jedes L dasselbe oder verschieden ist, wenn (m) gleich oder größer als 2 ist; und
(n) eine positive Ganzzahl ist;
vorausgesetzt dass R₁, R₂, R₃ und R₄ nicht alle OH sind;
oder ein pharmazeutisch akzeptables Salz davon.

2. Verbindung zur Verwendung in einem Verfahren zur Behandlung nach Anspruch 1, wobei (m) von 1 bis 10 ist, optional wobei (m) 1 ist.

3. Verbindung zur Verwendung in einem Verfahren zur Behandlung nach Anspruch 1, wobei (n) von 28 bis 341 ist, sodass das gesamte Molgewicht des Polymeranteils der Verbindung im Bereich von 5.000 bis 60.000 Dalton liegt, optional wobei (n) von 114 bis 239 ist, sodass das gesamte Molgewicht des Polymeranteils der Verbindung im Bereich von 20.000 bis 42.000 Dalton liegt, ferner optional wobei (n) 227 ist, sodass das gesamte Molgewicht des Polymeranteils der Verbindung 40.000 Dalton ist.

4. Verbindung zur Verwendung in einem Verfahren zur Behandlung nach Anspruch 1, wobei R₁, R₂, R₃ und R₄ alle 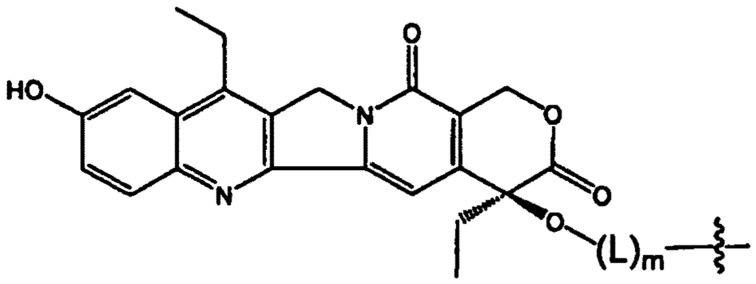 sind.

5. Verbindung zur Verwendung in einem Verfahren zur Behandlung nach Anspruch 1, wobei die Verbindung der Formel (I) aus der Gruppe, bestehend aus 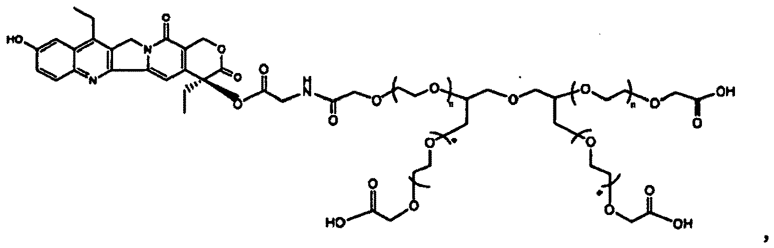 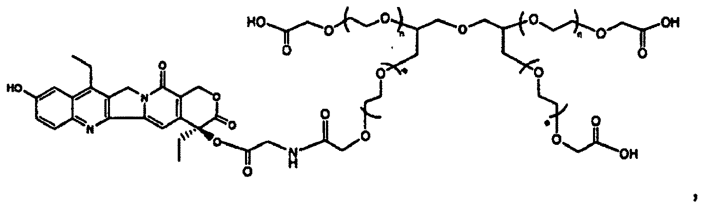 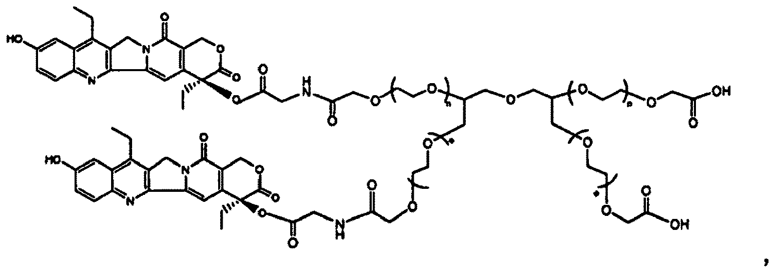 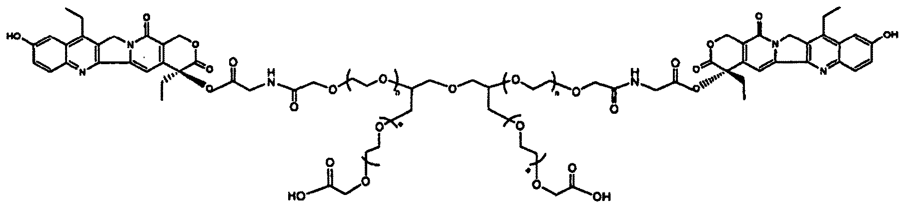 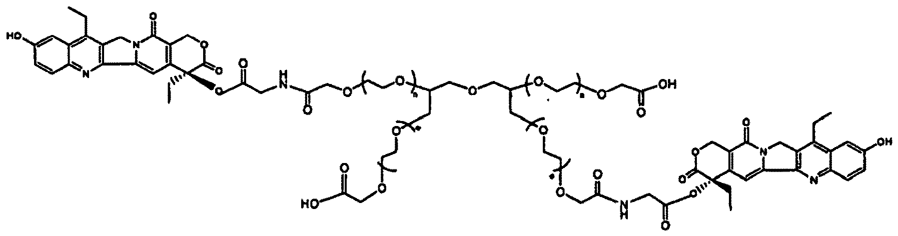 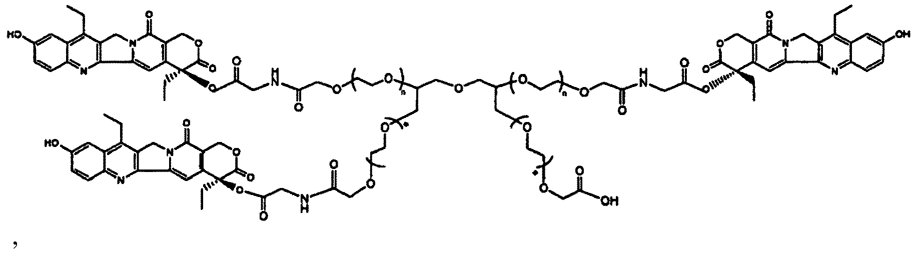 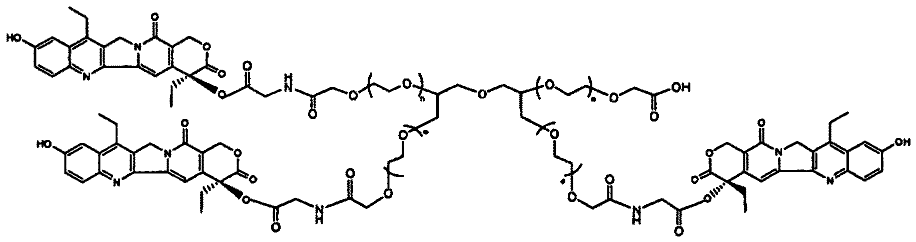 und 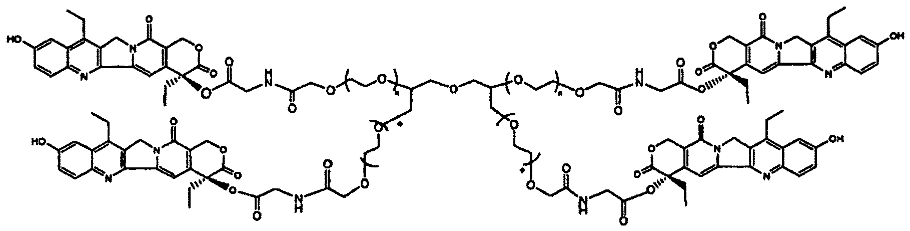 ausgewählt ist.

6. Verbindung zur Verwendung in einem Verfahren zur Behandlung nach Anspruch 1, wobei das Konjugat der Formel (I) in Mengen von 0,5 mg/m² Körperoberfläche/Dosis bis 50 mg/m² Körperoberfläche/Dosis verabreicht wird und wobei die Menge das Gewicht von 7-Ethyl-10-Hydroxycamptothecin, enthalten in der Verbindungder Formel (I), ist, optional in Mengen von 1 mg/m² Körperoberfläche/Dosis bis 18 mg/m² Körperoberfläche/Dosis und wobei die Menge das Gewicht von 7-Ethyl-10-Hydroxycamptothecin, enthalten in der Verbindung der Formel (I), ist.

7. Verbindung zur Verwendung in einem Verfahren zur Behandlung nach Anspruch 1, wobei die Verbindung der Formel (I) gemäß einem Protokoll von 1,25 mg/m² Körperoberfläche/Dosis bis 16,5 mg/m² Körperoberfläche/Dosis verabreicht wird, wöchentlich gegeben innerhalb drei Wochen, gefolgt von 1 Woche ohne Behandlung, und die Menge das Gewicht von 7-Ethyl-10-Hydroxycamptothecin, enthalten in der Verbindung der Formel (I), ist, optional wobei die wöchentlich verabreichte Menge 5 mg/m² Körperoberfläche/Dosis beträgt und die Menge das Gewicht von 7-Ethyl-10-Hydroxycamptothecin, enthalten in der Verbindung der Formel (I), ist.

8. Verbindung zur Verwendung in einem Verfahren zur Behandlung nach Anspruch 1, wobei der Krebs metastasierend ist, optional wobei der Krebs ein solider Tumor ist.

9. Verbindung zur Verwendung in einem Verfahren zur Behandlung nach Anspruch 1, wobei die Verbindung der Formel (I) in Kombination mit einem zweiten chemotherapeutischen Wirkstoff gleichzeitig oder nacheinander verabreicht wird, ferner optional wobei die Verbindung der Formel (I) verabreicht wird, gefolgt von 13-Cis-Retinolsäure.

10. Verbindung zur Verwendung in einem Verfahren zur Behandlung nach Anspruch 1, wobei die Verbindung der Formel (I) in Kombination mit Röntgentherapie gleichzeitig oder nacheinander verabreicht wird.

11. Verbindung zur Verwendung in einem Verfahren zur Behandlung nach Anspruch 1, wobei der Krebs resistent oder refraktär gegen eine Anti-Krebs-Therapie, die keine Verbindung der Formel (I) enthält, ist, und optional wobei der Krebs resistent gegen einen Anti-Krebs-Wirkstoff ist, ausgewählt aus Camptothecin, CPT-11, einem EGFR (Epidermal Growth Factor Receptor)-Antagonisten und Kombinationen davon, und ferner optional wobei der EGFR-Antagonist Cetuximab ist.

12. Verbindung der Formel: 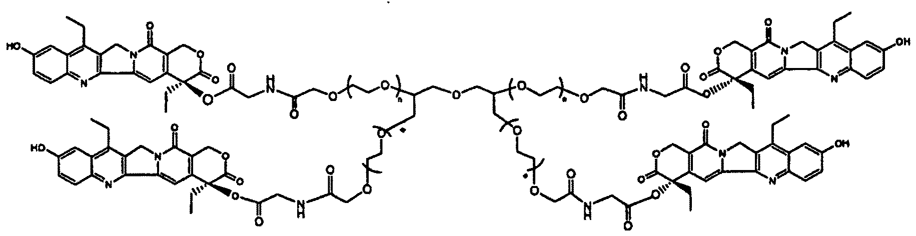 oder ein pharmazeutisch akzeptables Salz davon, zur Verwendung in einem Verfahren zur Behandlung von Neuroblastomen bei einem Säuger, der dieses benötigt, umfassend die Verabreichung
der Verbindung in Mengen von 1 mg/m² Körperoberfläche/Dosis bis 18 mg/m² Körperoberfläche/Dosis an den Säuger und die Menge ist das Gewicht von 7-Ethyl-Hydroxycamptothecin, enthalten in dem Konjugat,; und
(n) ist 227.

13. Verbindung zur Verwendung in einem Verfahren zur Behandlung nach Anspruch 12, wobei die Verbindung verabreicht wird gemäß einem Protokoll von 1,25 mg/m² Körperoberfläche/Dosis bis 16,5 mg/m² Körperoberfläche/Dosis, wöchentlich gegeben innerhalb drei Wochen, gefolgt von 1 Woche ohne Behandlung; und die Menge das Gewicht von 7-Ethyl-10-Hydroxycamptothecin, enthalten in der gegeben, ist, optional wobei die wöchentlich verabreichte Menge 5 mg/m² Körperoberfläche/Dosis ist und die Menge das Gewicht von 7-Ethyl-10-Hydroxycamptothecin, enthalten in der gegeben, ist.

14. Verbindung zur Verwendung in einem Verfahren zur Behandlung nach Anspruch 1, wobei L eine Aminosäure oder ein Aminosäurederivat ist, wobei das Aminosäurederivat ausgewählt ist aus der Gruppe bestehend aus 2-Aminoadipinsäure,3-Aminoadipinsäure, Beta-Alanin, Beta-Aminopropionsäure, 2-Aminobuttersäure, 4-Aminobuttersäure, Piperidinsäure, 6-Aminocapronsäure, 2-Aminoheptansäure, 2-Aminoisobuttersäure, 3-Aminoisobuttersäure, 2-Aminopimelinsäure, 2,4-Aminobuttersäure, Desmosin, 2,2-Diaminopimelinsäure, 2,3-Diaminopropionsäure, N-Ethylglycin, N-Ethylasparagin, 3-Hydroxyprolin, 4-Hydroxyprolin, Isodesmosin, Allo-Isoleucin, N-Methylglycin, Sarcosin, N-Methylisoleucin, 6-N-Methyllysin, N-Methylvalin, Norvalin, Norleucin und Ornithin, optional wobei L Glycin, Alanin, Methionin oder Sarcosin ist.

15. Verbindung zur Verwendung in einem Verfahren zur Behandlung nach Anspruch 1, wobei L ausgewählt ist aus der Gruppe bestehend aus
**-[C(=O)]ᵥ(CR₂₂R₂₃)ₜ-,**
**-[C(=O)]ᵥ(CR₂₂R₂₃)ₜ-O-,**
**-[C(=O)]ᵥ(CR₂₂R₂₃)ₜ-NR₂₆- ,**
**-[C(=O)]ᵥO(CR₂₂R₂₃)ₜ- ,**
**-[C(O)]ᵥO(CR₂₂R₂₃)ₜO- ,**
**-[C(=O)]ᵥO(CR₂₂R₂₃)ₜNR₂₆- ,**
**-[C(=O)]ᵥNR₂₁(CR₂₂R₂₃)ₜ- ,**
**-[C(=O)]ᵥNR₂₁(CR₂₂R₂₃)ₜO- ,**
**-[C(=O)]ᵥNR₂₁(CR₂₂R₂₃)ₜNR₂₆- ,**
**-[C(=O)]ᵥ(CR₂₂R₂₃O)ₜ- ,**
**-[C(=O)]ᵥO(CR₂₂R₂₃O)ₜ- ,**
**-[C(=O)]ᵥNR₂₁(CR₂₂R₂₃O)ₜ- ,**
**-[C(=O)]ᵥ(CR₂₂R₂₃O)ₜ(CR₂₄R₂₅)_{y}- ,**
**-[C(=O)]ᵥO(CR₂₂R₂₃O)ₜ(CR₂₄R₂₆)_{y}- ,**
**-[C(=O)]ᵥNR₂₁(CR₂₂R₂₃O)ₜ(CR₂₄R₂₅)_{y}- ,**
**-[C(=O)]ᵥ(CR₂₂R₂₃O)ₜ(CR₂₄R₂₅)_{y}O-,**
**-[C(=O)]ᵥ(CR₂₂R₂₃)ₜ(CR₂₄R₂₅O)_{y}-,**
**-[C(=O)]ᵥO(CR₂₂R₂₃O)ₜ(CR₂₄R₂₅)_{y}O-,**
**-[C(=O)]ᵥO(CR₂₂R₂₃)ₜ(CR₂₄R₂₅O)_{y}-,**
**-[C(=O)]ᵥNR₂₁(CR₂₂R₂₃O)ₜ(CR₂₄R₂₅)_{y}O-,**
**-[C(=O)]ᵥNR₂₁(CR₂₂R₂₃)ₜ(CR₂₄R₂₅O)_{y}-,**
**-[C(=O)]ᵥ(CR₂₂R₂₃)ₜO-(CR₂₈R₂₉)_{t'}-,**
**-[C(=O)]ᵥ(CR₂₂R₂₃)ₜNR₂₆-(CR₂₈R₂₉)_{t'}-,**
**-[C(=O)]ᵥ(CR₂₂R₂₃)ₜS-(CR₂₈R₂₉)_{t'}-,**
**-[C(=O)]ᵥO(CR₂₂R₂₃)ₜO-(CR₂₈R₂₉)_{t'}-,**
**-[C(=O)]ᵥO(CR₂₂R₂₃)ₜNR₂₆-(CR₂₈R₂₉)_{t'}-,**
**-[C(=C)]ᵥO(CR₂₂R₂₃)ₜS-(CR₂₈R₂₉)_{t'}-,**
**-[C(=O)]ᵥNR₂₁(CR₂₂R₂₃)ₜO-(CR₂₈R₂₉)_{t'}-,**
**-[C(=O)]ᵥNR₂₁(CR₂₂R₂₃)ₜNR₂₆-(CR₂₈R₂₉)_{t'}-,**
**-[C(=O)]ᵥNR₂₁(CR₂₂R₂₃)ₜS-(CR₂₈R₂₉)_{t'}-,**
**-[C(=O)]ᵥ(CR₂₂R₂₃CR₂₈R₂₉O)ₜNR₂₆-,**
**-[C(=O)]ᵥ(CR₂₂R₂₃CR₂₈R₂₉O)ₜ-,**
**-[C(=O)]ᵥO(CR₂₂R₂₃CR₂₈R₂₉O)ₜNR₂₆-,**
**-[C(=O)]ᵥO(CR₂₂R₂₃CR₂₈R₂₉O)ₜ-,**
**-[C(=O)]ᵥNR₂₁(CR₂₂R₂₃CR₂₈R₂₉O)ₜNR₂₆-,**
**-[C(=O)]ᵥNR₂₁(CR₂₂R₂₃CR₂₈R₂₉O)ₜ-,**
**-[C(=O)]ᵥ(CR₂₂R₂₃CR₂₈R₂₉O)ₜ(CR₂₄R₂₅)_{y}-,**
**-[C(=O)]ᵥO(CR₂₂R₂₃CR₂₈R₂₉O)ₜ(CR₂₄R₂₅)_{y}-,**
**-[C(=O)]ᵥNR₂₁(CR₂₂R₂₃CR₂₈R₂₉O)ₜ(CR₂₄R₂₅)_{y}-,**
**-[C(=O)]ᵥ(CR₂₂R₂₃CR₂₈R₂₉O)ₜ(CR₂₄R₂₅)_{y}O-,**
**-[C(=(O)]ᵥ(CR₂₂R₂₃)ₜ(CR₂₄R₂₅CR₂₈R₂₉O)_{y}-,**
**-[C(=O)]ᵥ(CR₂₂R₂₃)ₜ(CR₂₄R₂₅CR₂₈R₂₉O)_{y}NR₂₆-,**
**-[C(=O)]ᵥO(CR₂₂R₂₃CR₂₈R₂₉O)ₜ(CR₂₄R₂₅)_{y}O-,**
**-[C(=O)]ᵥO(CR₂₂R₂₃)ₜ(CR₂₄R₂₅CR₂₈R₂₉O)_{y}-,**
**-[C(=O)]ᵥO(CR₂₂R₂₃)ₜ(CR₂₄CR₂₅CR₂₈R₂₉O)_{y}NR₂₆-,**
**-[C(=O)]ᵥNR₂₁(CR₂₂R₂₃CR₂₈R₂₉O)ₜ(CR₂₄R₂₅)_{y}O-,**
**-[C(=O)]ᵥNR₂₁(CR₂₂R₂₃)ₜ(CR₂₄R₂₅CR₂₈R₂₉O)_{y}-,**
**-[C(=)]ᵥNR₂₁(CR₂₂R₂₃)ₜ(CR₂₄R₂₅CR₂₈R₂₉O)_{y}NR₂₆-,** 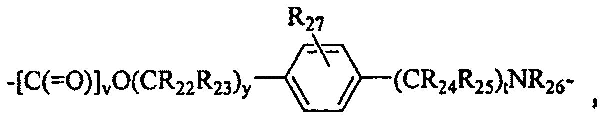 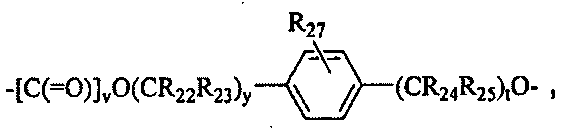 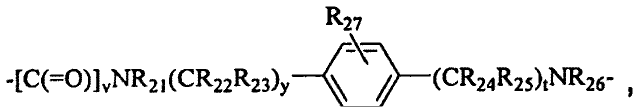 und 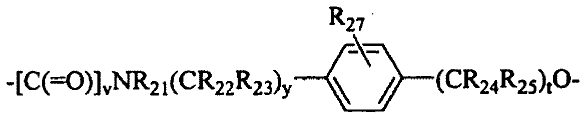 wobei
R₂₁-R₂₉ unabhängig ausgewählt sind aus der Gruppe bestehend aus Wasserstoff, Amino, substituiertem Amino, Azido, Carboxy, Cyano, Halo, Hydroxyl, Nitro, Silylether, Sulfonyl, Mercapto, C₁₋₆ Alkylmercapto, Arylmercapto, substituiertem Arylmercapto, substituiertem C₁₋₆ Alkylthio, C₁₋₆ Alkylen, C₂₋₆ Alkenyl, C₂₋₆ Alkynyl, C₃₋₁₉ verzweigtem Alkyl, C₃₋₈ Cycloalkyl, C₁₆ substituiertem Alkyl, C₂₋₆ substituiertem Alkenyl, C₂₋₆ substituiertem Alkynyl, C₃₋₈ substituiertem Cycloalkyl, Aryl, substituiertem Aryl, Heteroaryl, substituiertem Heteroaryl, C₁₋₆ Heteroalkyl, substituiertem C₁₋₆ Heteroalkyl, C₁₋₆ Alkoxy, Aryloxy, C₁₋₆ Heteroalkoxy, Heteroaryloxy, C₂₋₆ Alkanoyl, Arylcarbonyl, C₂₋₆ Alkoxycarbonyl, Aryloxycarbonyl, C₂₋₆ Alkanoyloxy, Arylcarbonyloxy, C₂₋₆ substituiertem Alkanoyl, substituiertem Arylcarbonyl, C₂₋₆ substituiertem Alkanoyloxy, substituiertem Aryloxycarbonyl, C2-6 substituiertem Alkanoyloxy, substituiertem und Arylcarbonyloxy;
(t), (t') und (y) unabhängig ausgewählt sind aus Null oder einer positiven Ganzzahl; und
(v) 0 oder 1 ist.

## Revendications

1. Composé de formule (I) destiné à être utilisé dans un procédé de traitement du neuroblastome comprenant l'administration du composé de formule (I) à un mammifère qui en a besoin, où le composé de formule (I) comprend : 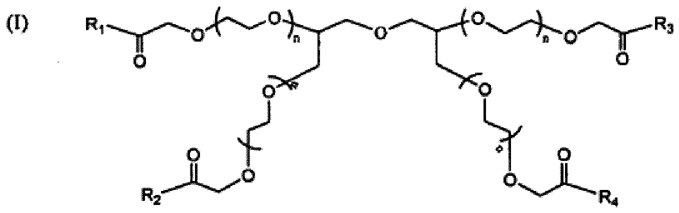 où
R₁, R₂, R₃ et R₄ sont indépendamment OH, ou 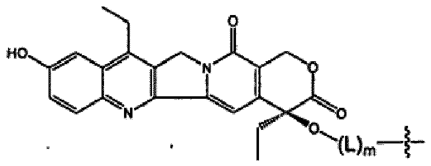 où
L est un lieur bifonctionnel ;
(m) est un entier positif, où chaque L est identique ou différent quand (m) est égal ou supérieur à 2 ; et
(n) est un entier positif ;
à condition que R₁, R₂, R₃ et R₄ ne soient pas tous OH ;
ou sel pharmaceutiquement acceptable de celui-ci.

2. Composé destiné à être utilisé dans un procédé pour le traitement selon la revendication 1, où (m) est de 1 à 10, éventuellement où (m) est 1.

3. Composé destiné à être utilisé dans un procédé pour le traitement selon la revendication 1, où (n) est de 28 à 341 de sorte que la masse moléculaire totale de la partie polymère du composé va de 5 000 à 60 000 daltons, éventuellement où (n) est de 114 à 239 de sorte que la masse moléculaire totale de la partie polymère du composé va de 20 000 à 42 000 daltons, éventuellement en outre où (n) est 227 de sorte que la masse moléculaire totale de la partie polymère du composé est 40 000 daltons.

4. Composé destiné à être utilisé dans un procédé pour le traitement selon la revendication 1, où R₁, R₂, R₃ et R₄ sont tous :

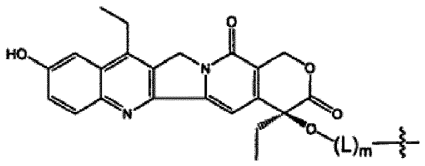

5. Composé destiné à être utilisé dans un procédé pour le traitement selon la revendication 1, où le composé de formule (I) est choisi dans le groupe consistant en 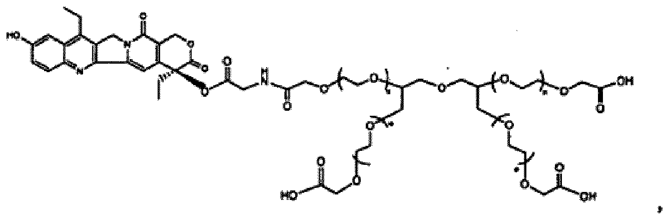 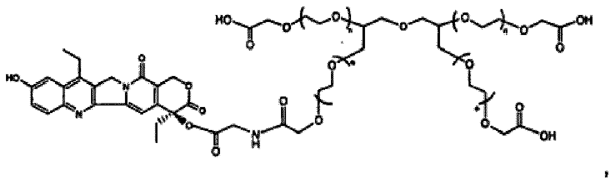 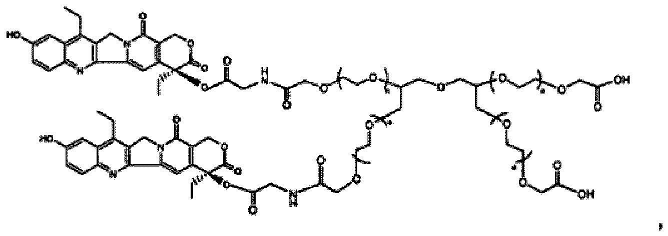 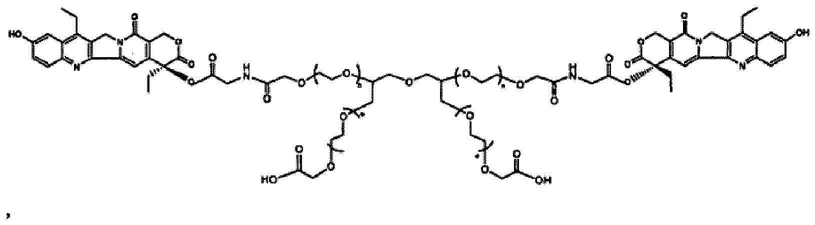 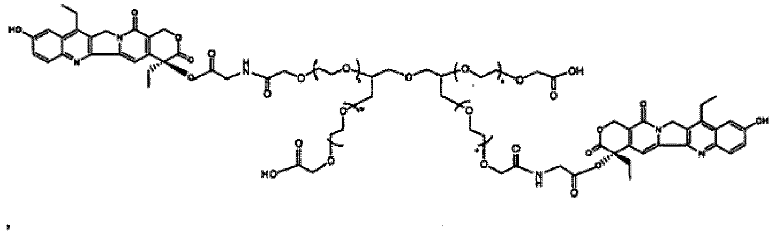 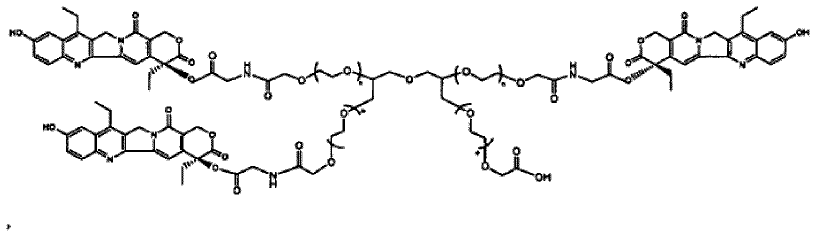 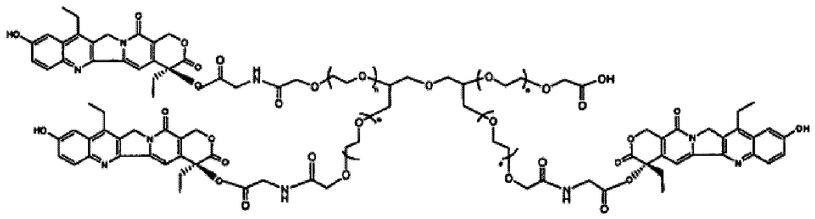 et

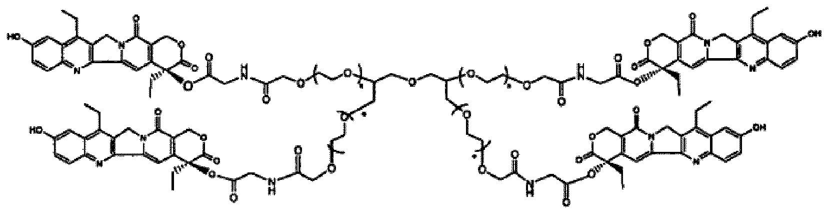

6. Composé destiné à être utilisé dans un procédé pour le traitement selon la revendication 1, où le composé de formule (I) est administré en des quantités de 0,5 mg/m² de surface corporelle/dose à 50 mg/m² de surface corporelle/dose, et où la quantité est le poids de 7-éthyl-10-hydroxycamptothécine inclus dans le composé de formule (I), éventuellement en des quantités de 1 mg/m² de surface corporelle/dose à 18 mg/m² de surface corporelle/dose, et où la quantité est le poids de 7-éthyl-10-hydroxycamptothécine inclus dans le composé de formule (I).

7. Composé destiné à être utilisé dans un procédé pour le traitement selon la revendication 1, où le composé de formule (I) est administré selon un protocole de 1,25 mg/m² de surface corporelle/dose à 16,5 mg/m² de surface corporelle/dose donné toutes les semaines pendant trois semaines, suivi par une semaine sans traitement, et la quantité est le poids de 7-éthyl-10-hydroxycamptothécine inclus dans le composé de formule (I), éventuellement où la quantité administrée toutes les semaines est 5 mg/m² de surface corporelle/dose, et la quantité est le poids de 7-éthyl-10-hydroxycamptothécine inclus dans le composé de formule (I).

8. Composé destiné à être utilisé dans un procédé pour le traitement selon la revendication 1, où le cancer est métastatique, éventuellement où le cancer est une tumeur solide.

9. Composé destiné à être utilisé dans un procédé pour le traitement selon la revendication 1, où le composé de formule (I) est administré en combinaison avec un second agent chimiothérapeutique simultanément ou successivement, éventuellement en outre où le composé de formule (I) est administré, suivi par l'acide 13-cis-rétinoïque.

10. Composé destiné à être utilisé dans un procédé pour le traitement selon la revendication 1, où le composé de formule (I) est administré en combinaison avec une radiothérapie simultanément ou successivement.

11. Composé destiné à être utilisé dans un procédé pour le traitement selon la revendication 1, où le cancer est résistant ou réfractaire à une thérapie anticancéreuse qui n'inclut pas un composé de formule (I), et éventuellement où le cancer est résistant à un agent anticancéreux qui est choisi parmi la camptothécine, CPT-11, un antagoniste de récepteur de facteur de croissance épidermique, et leurs combinaisons, et éventuellement en outre où l'antagoniste de récepteur de facteur de croissance épidermique est le cetuximab.

12. Composé ayant la formule : 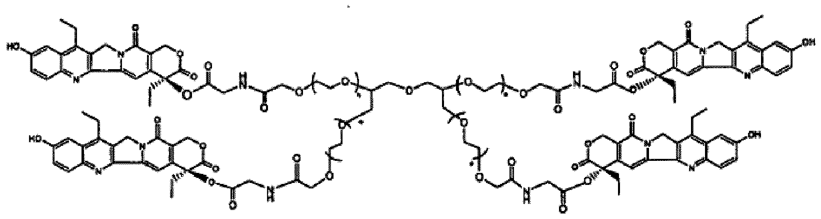 ou sel pharmaceutiquement acceptable de celui-ci, destiné à être utilisé dans un procédé de traitement du neuroblastome chez un mammifère qui en a besoin, comprenant l'administration audit mammifère
du composé en des quantités de 1 mg/m² de surface corporelle/dose à 18 mg/m² de surface corporelle/dose et la quantité est le poids de 7-éthyl-10-hydroxycamptothécine inclus dans le composé ; et
(n) est 227.

13. Composé destiné à être utilisé dans un procédé pour le traitement selon la revendication 12, où le composé est administré selon un protocole de 1,25 mg/m² de surface corporelle/dose à 16,5 mg/m² de surface corporelle/dose donné toutes les semaines pendant trois semaines, suivi par 1 semaine sans traitement ; et la quantité est le poids de 7-éthyl-10-hydroxycamptothécine inclus dans le composé, éventuellement où la quantité administrée toutes les semaines est 5 mg/m² de surface corporelle/dose, et la quantité est le poids de 7-éthyl-10-hydroxy-camptothécine inclus dans le composé.

14. Composé destiné à être utilisé dans un procédé pour le traitement selon la revendication 1, où L est un aminoacide ou un dérivé d'aminoacide, où le dérivé d'aminoacide est choisi dans le groupe consistant en acide 2-aminoadipique, acide 3-aminoadipique, bêta-alanine, acide bêta-aminopropionique, acide 2-aminobutyrique, acide 4-aminobutyrique, acide pipéridinique, acide 6-aminocaproïque, acide 2-aminoheptanoïque, acide 2-aminoisobutyrique, acide 3-aminoisobutyrique, acide 2-aminopimélique, acide 2,4-aminobutyrique, desmosine, acide 2,2-diaminopimélique, acide 2,3-diaminopropionique, N-éthylglycine, N-éthyl-asparagine, 3-hydroxyproline, 4-hydroxyproline, isodesmosine, allo-isoleucine, N-méthylgycine, sarcosine, N-méthyl-isoleucine, 6-N-méthyllysine, N-méthylvaline, norvaline, norleucine et ornithine, éventuellement où L est la glycine, l'alanine, la méthionine ou la sarcosine.

15. Composé destiné à être utilisé dans un procédé pour le traitement selon la revendication 1, où L est choisi dans le groupe consistant en
**-[C(=O)]ᵥ(CR₂₂R₂₃)ₜ-,**
**-[C(=O)]ᵥ(CR₂₂R₂₃)ₜ-O-,**
**-[C(=O)]ᵥ(CR₂₂R₂₃)ₜ-NR₂₆-,**
**-[C(=O)]ᵥO(CR₂₂R₂₃)ₜ-,**
**-[C(=O)]ᵥO(CR₂₂R₂₃)ₜO-,**
**-[C(=O)]ᵥO(CR₂₂R₂₃)ₜNR₂₆-,**
**-[C(=O)]ᵥNR₂₁(CR₂₂R₂₃)ₜ-,**
**-[C(=O)]ᵥNR₂₁(CR₂₂R₂₃)ₜO-,**
**-[C(=O)]ᵥNR₂₁(CR₂₂R₂₃)ₜNR₂₆-,**
**-[C(=O)]ᵥ(CR₂₂R₂₃O)ₜ-,**
**-[C(=O)]ᵥO(CR₂₂R₂₃O)ₜ-,**
**-[C(=O)]ᵥNR₂₁(CR₂₂R₂₃O)ₜ-,**
**-[C(=O)]ᵥ(CR₂₂R₂₃O)ₜ(CR₂₄R₂₅)_{y}-,**
**-(C(=O)]ᵥO(CR₂₂R₂₃O)ₜCR₂₄R₂₆)_{y}-,**
**-(C(=O)]ᵥNR₂₁(CR₂₂R₂₃O)ₜ(CR₂₄R₂₅)_{y-},**
**-[C(=O)]ᵥ(CR₂₂R₂₃O)ₜ(CR₂₄R₂₅)_{y}O-,**
**-[C(=O)]ᵥ(CR₂₂R₂₃)ₜ(CR₂₄R₂₅O)_{y}-,**
**-[C(=O)]ᵥO(CR₂₂R₂₃O)ₜ(CR₂₄R₂₅)_{y}O-,**
**-[C(=O)]ᵥO(CR₂₂R₂₃)ₜCR₂₄R₂₅O)_{y}-,**
**-[C(=O)]vNR₂₁(CR₂₂R₂₃O)ₜ(CR₂₄R₂₅)_{y}O-,**
**-[C(=O)]ᵥNR₂₁(CR₂₂R₂₃)ₜ(CR₂₄R₂₅O)_{y}-,**
**-[C(=O)]ᵥ(CR₂₂R₂₃)ₜO-(CR₂₈R₂₉)_{t'}-,**
**-[C(=O)]ᵥ(CR₂₂R₂₃)ₜNR₂₆-(CR₂₈R₂₉)_{t'}-,**
**-[C(=O)]ᵥ(CR₂₂R₂₃)ₜS-(CR₂₈R₂₉)_{t'},**
**-(C(=O)]ᵥO(CR₂₂R₂₃)ₜO-(CR₂₈R₂₉)_{t'}-,**
**-[C(=O)]ᵥO(CR₂₂R₂₃)ₜNR₂₆-(CR₂₈R₂₉)_{t'}-,**
**-[C(=O)]ᵥO(CR₂₂R₂₃)ₜS-(CR₂₈R₂₉)_{t'}-,**
**-[C(=O)]ᵥNR₂₁(CR₂₂R₂₃)ₜO-(CR₂₈R₂₉)_{t'}-,**
**-[C(=O)]ᵥNR₂₁(CR₂₂R₂₃)ₜNR₂₆-(CR₂₈R₂₉)_{t'}-,**
**-[C(=O)]ᵥNR₂₁(CR₂₂R₂₃)ₜS-(CR₂₈R₂₉)_{t'}-,**
**-[C(=O)]ᵥ(CR₂₂R₂₃CR₂₈R₂₉O)ₜNR₂₆-,**
**-[C(=O)]ᵥ(CR₂₂R₂₃CR₂₈R₂₉O)ₜ-,**
**-[C(=O)]ᵥO(CR₂₂R₂₃CR₂₈R₂₉O)ₜNR₂₆-,**
**-[C(=O)]ᵥO(CR₂₂R₂₃CR₂₈R₂₉O)ₜ-,**
**-[C(=O)]ᵥNR₂₁(CR₂₂R₂₃CR₂₈R₂₉O)ₜNR₂₆-,**
**-[C(=O)]ᵥNR₂₁(CR₂₂R₂₃CR₂₈R₂₉O)ₜ-,**
**-[C(=O)]ᵥ(CR₂₂R₂₃CR₂₈R₂₉O)ₜ(CR₂₄-R₂₅)_{y}-,**
**-[C(=O)]ᵥO(CR₂₂R₂₃CR₂₈R₂₉O)ₜ(CR₂₄R₂₅)_{y}-,**
**-[C(=O)]ᵥNR₂₁(CR₂₂R₂₃CR₂₈R₂₉O)ₜ(CR₂₄R₂₅)_{y}-,**
**-[C(=O)]ᵥ(CR₂₂R₂₃CR₂₈R₂₉O)ₜ(CR₂₄R₂₅)_{y}O-,**
**-[C(=O)]ᵥ(CR₂₂R₂₃)ₜ(CR₂₄R₂₅CR₂₈R₂₉O)_{y}-,**
**-[C(=O)]ᵥ(CR₂₂R₂₃)ₜ(CR₂₄R₂₅CR₂₈R₂₉O)_{y}NR₂₆-,**
**-[C(=O)]ᵥO(CR₂₂R₂₃CR₂₈R₂₉O)ₜ(CR₂₄R₂₅)_{y}O-,**
**-[C(=O)]ᵥO(CR₂₂R₂₃)ₜ(CR₂₄R₂₅CR₂₈R₂₉O)_{y}-,**
**-[C(=O)]ᵥO(CR₂₂R₂₃)ₜ(CR₂₄CR₂₅CR₂₈R₂₉O)_{y}NR₂₆-,**
**-[C(=O)]ᵥNR₂₁(CR₂₂R₂₃CR₂₈R₂₉O)ₜ(CR₂₄R₂₅)_{y}O-,**
**-[C(=O)]ᵥNR₂₁(CR₂₂R₂₃)ₜ(CR₂₄R₂₅CR₂₈R₂₉O)_{y}-,**
**-[C(=O)]ᵥNR₂₁(CR₂₂R₂₃)ₜ(CR₂₄R₂₅CR₂₈R₂₉O)_{y}NR₂₆-,** 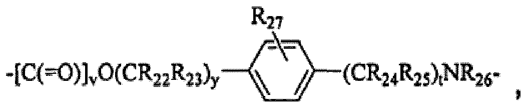 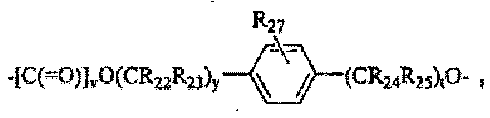 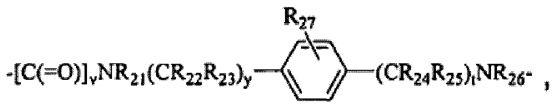 **et** 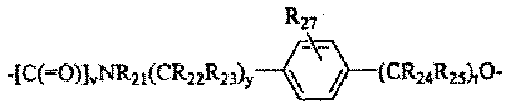 où :
R₂₁-R₂₉ sont choisis indépendamment dans le groupe consistant en hydrogène, amino, amino substitué, azido, carboxy, cyano, halo, hydroxyle, nitro, silyl éther, sulfonyle, mercapto, C₁₋₆-alkylmercapto, arylmercapto, arylmercapto substitué, C₁₋₆ alkylthio substitué, C₁₋₆ alkyle, C₂₋₆ alcényle, C₂₋₆ alcynyle, C₃₋₁₉ alkyle ramifié, C₃₋₈ cycloalkyle, C₁₋₆ alkyle substitué, C₂₋₆ alcényle substitué, C₂₋₆ alcynyle substitué, C₃₋₈ cycloalkyle substitué, aryle, aryle substitué, hétéroaryle, hétéroaryle substitué, C₁₋₆ hétéroalkyle, C₁₋₆ hétéroarlkyle substitué, C₁₋₆ alcoxy, aryloxy, C₁₋₆ hétéroalcoxy, hétéroaryloxy, C₂₋₆ alcanoyle, arylcarbonyle, C₂₋₆ alcoxycarbonyle, aryloxycarbonyle, C₂₋₆ alcanoyloxy, arylcarbonyloxy, C₂₋₆ alcanoyle substitué, arylcarbonyle substitué, C₂₋₆ alcanoyloxy substitué, aryloxycarbonyle substitué, C₂₋₆ alcanoyloxy substitué, et arylcarbonyloxy substitué ;
(t), (t') et (y) sont choisis indépendamment parmi zéro ou un entier positif ; et
(v) est 0 ou 1.
